(19) Europäisches Patentamt
European Patent Office
Office européen des brevets

(11) Publication number: **0 379 174 A2**

# (12) EUROPEAN PATENT APPLICATION

(21) Application number: **90100924.1**

(22) Date of filing: **17.01.90**

(51) Int. Cl.[5] **C07D 307/78, C07D 409/12, C07D 407/12, C07D 405/12, A01N 43/12**

A request for correction of "obvious errors" on pages 57-62 and 64-69 has been filed pursuant to Rule 88 EPC. A decision on the request will be taken during the proceedings before the Examining Division (Guidelines for Examination in the EPO, A-V, 2.2).

(30) Priority: **18.01.89 JP 7703/89**
**13.10.89 JP 265046/89**

(43) Date of publication of application:
**25.07.90 Bulletin 90/30**

(84) Designated Contracting States:
**DE ES FR GB IT**

(71) Applicant: **MITSUI TOATSU CHEMICALS, Inc.**
**2-5 Kasumigaseki 3-chome**
**Chiyoda-Ku Tokyo 100(JP)**

(72) Inventor: **Asano, Tamotsu**
**1071-2, Nakano-cho, Sakae-ku**
**Yokohama-shi, Kanagawa-ken(JP)**
Inventor: **Ozawa, Shuji**
**2882, Iijima-cho, Sakae-ku**
**Yokohama-shi, Kanagawa-ken(JP)**
Inventor: **Arai, Kiyoshi**
**2882, Iijima-cho, Sakae-ku**
**Yokohama-shi, Kanagawa-ken(JP)**
Inventor: **Yamazaki, Hideo**
**1937-7, Kamiiida-cho, Izumi-ku**
**Yokohama-shi, Kanagawa-ken(JP)**
Inventor: **Miura, Tohru**
**1612, Kosugaya-cho, Sakae-ku**
**Yokohama-shi, Kanagawa-ken(JP)**
Inventor: **Gohbara, Masatoshi**
**Nakano Village 301, 1071-2, Nakano-cho, Sakae-ku**
**Yokohama-shi, Kanagawa-ken(JP)**
Inventor: **Oyamada, Masami**
**2787, Honnou**
**Mobara-shi, Chiba-ken(JP)**
Inventor: **Nishida, Makoto**
**2882, Iijima-cho, Sakae-ku**
**Yokohama-shi, Kanagawa-ken(JP)**

(74) Representative: **Zumstein, Fritz, Dr. et al**
**Dr. F. Zumstein Dipl.-Ing. F. Klingseisen**
**Bräuhausstrasse 4**
**D-8000 München 2(DE)**

## (54) 2,3-Dihydro-3,3-dimethyl-5-trifluoromethanesulfonyloxybenzofurans, process for producing them and herbicides containing them.

(57) Disclosed are novel 2,3-dihydro-3,3-dimethyl-5-trifluoromethanesulfonyloxy benzofurans and herbicides containing them, which are produced, e.g., via the mono-substituted 2-morpholino derivative, by reacting a corresponding 5-hydroxy benzofuran with a trifluoromethanesulfonyl esterifying agent.

EP 0 379 174 A2

# 2,3-DIHYDRO-3,3-DIMETHYL-5-TRIFLUOROMETHANE-SULFONYLOXY-BENZOFURANS, PROCESS FOR PRODUCING THEM AND HERBICIDES CONTAINING THEM

## BACKGROUND OF THE INVENTION

### Field of the Invention

This invention relates to novel 2,3-dihydrobenzofuran derivatives and herbicides containing them as a herbicidally effective ingredinet.

### Description of the Prior Art

It has been reported that alkyl sulfonate derivatives of 2,3-dihydrobenzofuran-5-ols have herbicidal activity in, for example, USP-3,689,507, USP- 3,937,702, USP-4,162,154, and USP-4,014,904. The compounds described in these publications are used as upland herbicides and there are no test examples of their use as herbicides for rice plants in paddy field.

In view of the above, the present inventors have tested and evaluated the compounds described in these publications in paddy field and, as a result, have found that they show insufficient herbicidal acitivity to barnyardgrass which is one of most important weeds in paddy field, as well as involve a basic drawback that they cause serious phytotoxicity to rice plants in paddy field as crops.

The present inventors have made various studies on the herbicidal activity of 2,3-dihydrobenzofuran series compounds and, as a result, have found that the compounds of this series of the prior art have an insufficient herbicidal activity to barnyardgrass, which is one of the most important weeds in paddy field and they have the basic defect that they show serious phytotoxicity to rice plants in paddy field as crops.

It is, accordingly, an object of the present invention to provide a compound that can be applied in upland, as well as paddy field, giving no phytotoxicity to rice plants in paddy field and exhibiting excellent herbicidal activity.

A specific object of the present invention is to provide a compound not deleterious to rice plants in paddy field and showing strong herbicidal activity against annual weeds such as Echinochloa crus-galli, Cyperus difformis, Monochroria vaginalis and Ratala indica as well as perennial weeds such as Scirpus juncoides, Cyperus serotinus, Eleocharis kuroguwai, Eleocharis acicularis and Sagittaria pygmaea, thus having excellent performance as a paddy field rice herbicide.

Another object of the present invention is to provide a compound which is effective, through preemergence treatment or postemergence treatment, against one or more of Digitaria sanguinalis, Stellaria media, Polygonum lapathifolium, Amaranthus lividus, Cyperus iria, Portulaca oleracea, Senecio vulgaris, Chenopodium album, Cyperus rotundus, Calystegia sepium, Sagina japonica, Galium spurium, Alopecurus aequalis, Pea annua, Capsella bursa-pastoris and Setaria viridis, and also effective as other non-agricultural land herbicide.

## SUMMARY OF THE INVENTION

For achieving the foregoing objects, the present inventors have made an earnest study on herbicidal activity of 2,3-dihydrofuran series compounds and have accomplished the present invention based on the finding of a group of compounds which are effective not only as a herbicide for paddy field rice plants but also as herbicides for upland and other non-agricultural land.

The present invention provides in general the herbicidally active 2,3-dihydro-3,3-dimethyl-5-trifluoromethanesulfonyloxy benzofurans and the corresponding benzofurans substituted or disubstituted in the 2-position and/or in the 7-position by a simple, non-interfering substituent, and herbicidal compositions comprising one or more thereof. In a preferred aspect, the 2,3-dihydrobenzofuran derivatives are represented by the general formula (I):

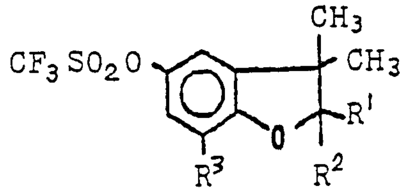

( I )

wherein $R^1$ represents a hydrogen atom, hydroxy, lower alkoxy, amino ($-NH_2$), morpholino or an esterified hydroxy group, e.g., of the formula $-OCOR^4$ ( wherein $R^4$ represents lower alkyl, cycloalkyl, halogen-substituted lower alkyl, lower alkoxy-substituted lower alkyl, or alkoxycarbonylalkyl, e.g., of the formula $-(CH_2)_nCO_2R^5$ wherein $R^5$ represents lower alkyl and n represents the integer of 1 or 2), benzyl, styryl, lower alkoxy, phenoxy, pyridyl, thienyl, furyl, phenyl or substituted phenyl, e.g., of the formula

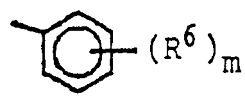

(wherein $R^6$ represents a hydrogen atom,halogne atom, cyano, acetoxy, lower alkyl, lower alkoxy and m represents an integer from 1 to 5); $R^2$ represents a hydrogen atom or together with $R^1$ represents an oxygen atom, and $R^3$ represents a hydrogen atom, methyl or a halogen atom. In other aspects this invention relates to a process for producing compounds of this invention and to herbicidal compositions containing one or more of the compounds of this invention as a herbicidally effective ingredient.

Examples of compounds within Formula (I) are those wherein

a) at least one of $R^1$, $R^2$ and $R^3$, preferably at least $R^2$, e.g., $R^1$ and $R^2$, is a hydrogen atom;

b) $R^3$ is lower alkyl, preferably $CH_3$, e.g., those wherein one or more of $R^1$ and $R^2$ is a hydrogen atom; and

c) wherein $R^1$ is an esterified hydroxy group, morpholino, hydroxy or collectively with $R^2$ an oxygen atom, e.g., those wherein $R^3$ is lower alkyl.

Specific examples for $R^1$ in the general formula (I) are a hydrogen atom, hydroxy, lower alkoxy such as methoxy, ethoxy, iso-propoxy, n-propoxy, n-butoxy, iso-butoxy, sec-butoxy and t-butoxy, amino, morpholino, an acyloxy group such as acetoxy, propionyloxy, butyryloxy, isobutyryloxy, pivaloyloxy, decanoyloxy, phenylacetoxy, cyclohexane carbonyloxy, chloroacetoxy, dichloroacetoxy, trichloroacetoxy, α-chloropropionyloxy, methoxyacetoxy, benzoyloxy, p-methylbenzoyloxy, m-methylbenzoyloxy, o-methylbenzoyloxy, p-t-butylbenzoyloxy, o-chlorobenzoyloxy, m-chlorobenzoyloxy, p-chlorobenzoyloxy, o-fluorobenzoyloxy, m-fluorobenzoyloxy, p-fluorobenzoyloxy, o-bromobenzoyloxy, m-bromobenzoyloxy, p-bromobenzoyloxy, 2,4-dichlorobenzoyloxy, 2,6-dichlorobenzoyloxy, 3,4-dichlorobenzoyloxy, pentafluorobenzoyloxy, p-methoxybenzoyloxy, 3,4,5-trimethoxybenzoyloxy, m-methoxybenzoyloxy, o-methoxybenzoyloxy, p-trifluoromethylbenzoyloxy, p-nitrobenzoyloxy, p-cyanobenzoyloxy, o-acetoxybenzoyloxy, 2-thienyloxy, 3-thienyloxy, 2-furoyloxy, 3-furoyloxy, pyridin-2-ylcarbonyloxy, and pyridin-3-ylcarbonyloxy, cinnamoyloxy, ethoxycarbonylacetoxy, β-(methoxycarbonyl) propionyloxy and, an alkoxycarbonyloxy group such as methoxycarbonyloxy, ethoxycarbonyloxy, n-propoxycarbonyloxy, n-butoxycarbonyloxy and phenoxycarbonyloxy. Specific examples of $R^3$ are a hydrogen atom, methyl group, chlorine atom, bromine atom and fluorine atom.

Production process for the compounds according to the present invention will now be described.

The compounds according to the present invention except for those in which both of $R^1$ and $R^2$ are hydrogen atoms in the general formula (I) can be produced by known methods as described, for example, in Journal of Organic Chemistry, vol. 33, p3346 (1968), USP-3,689,507, USP-3,937,702 and USP-3,184,457, as well at those methods according to them. That is, they can be produced by the steps shown below (Synthesis Route A)) starting from the compound represented by the general formula (III) obtained by reacting a benzoquinone derivative having a desired substituent and an enamine form of isopropylaldehyde and morpholine.

Synthesis Route (A)

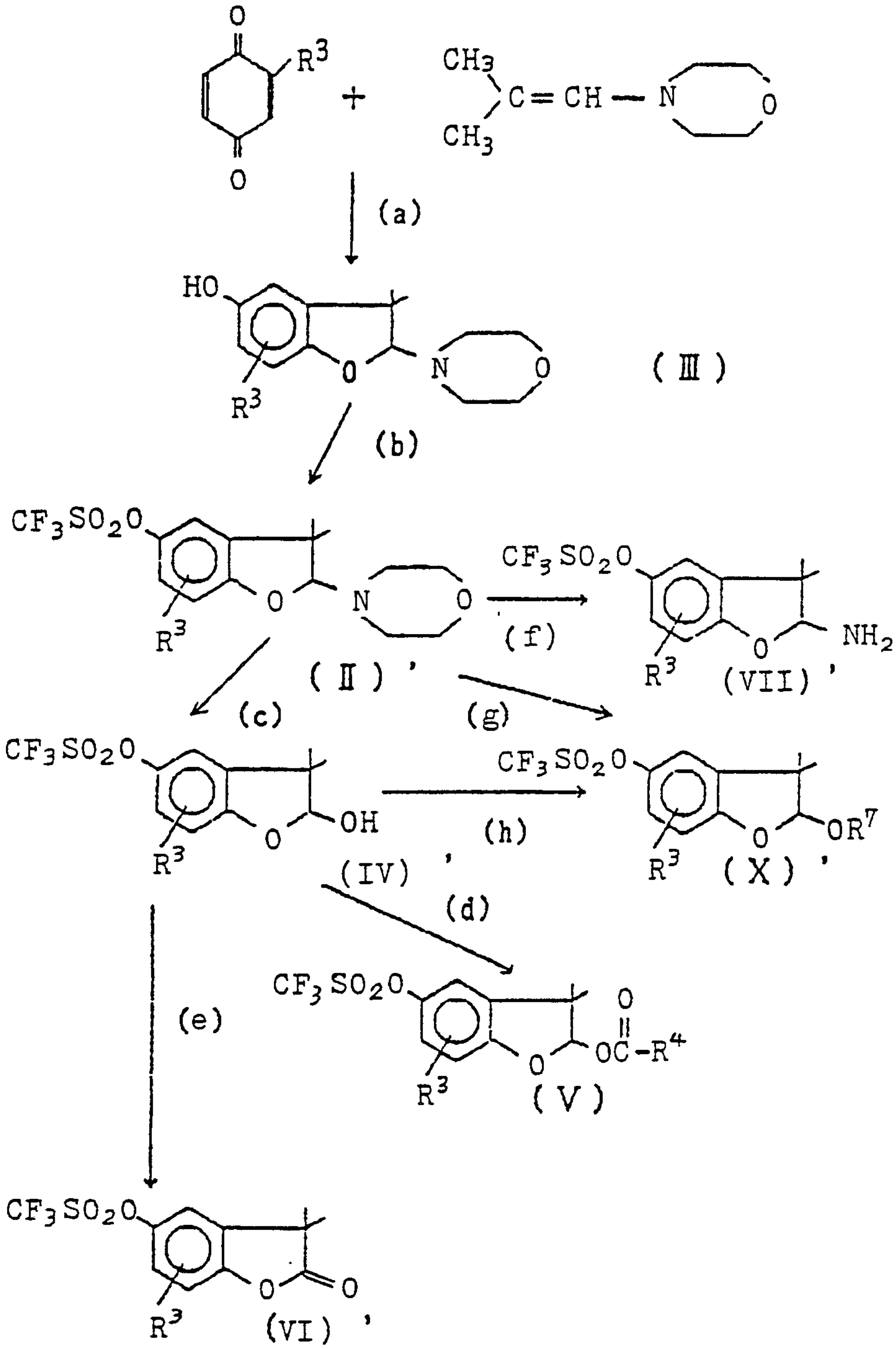


(III)

(II)', (VII)', (IV)', (X)', (V), (VI)'

where $R^3$ and $R^4$ have the same meanings as described above and $R^7$ represents a lower alkyl group.

That is, the compound represented by the general formula (III) is obtained from the benzoquinone derivative and the enamine form through the step (a). In a case where $R^3$ is other than a hydrogen atom, the aimed compound is obtained, generally, as a mixture of those having 6-substituent and 7-substituent.

The compound represented by the general formula (II)′ is prepared through the step (b) in an organic solvent by dissolving the compound represented by the general formula (III) in an inert solvent such as benzene, toluene, xylene, chlorobenzene, diethyl ether, dioxane, tetrahydrofuran, methylene chloride, chloroform, methyl ethyl ketone and acetone, and brought into reaction with addition of an equi-molar amount or a slight excess of trifluoromethane sulfonyl halide or trifluoromethane sulfonic acid anhydride and an organic base such as triethylamine or pyridine, dimethylaniline or inorganic powderized carbonate such as potassium carbonate or sodium carbonate. Further, the reaction may be conducted by using metal sodium or sodium hydride, or pyridine may be used also as a reaction solvent. The reaction temperature

can be from -50 °C to the boiling point of the solvent and it is advantageous to carry out the reaction at a relatively low temperature range. After the reaction is over, usual post-treatment is applied and the aimed compound can be purified by means of recrystallization or column chromatography.

The compound represented by the general formula (IV)′ can be obtained through the step (c) by hydrolysis usually in the aqueous solvent by using hydrochloric acid or sulfuric acid as a catalyst.

The compound represented by the general formula (X)′ can be obtained through the step (g) by reacting a compound represented by the formula (II)′ in a corresponding alcohol under the presence of a catalyst such as sulfuric acid or hydrochloric acid, or through the step (h) by etherifying via dehydration the compound represented by the formula (II)′ in a corresponding alcohol under the presence of a sulfuric acid catalyst.

The compound represented by the general formula (V)′ can be obtained through the step (d) by reacting a compound represented by the general formula (IV)′ in an inert solvent such as n-hexane, benzene, toluene, xylene, diethyl ether, dioxane, tetrahydrofuran, methylene chloride, chloroform, methyl ethyl ketone, acetone and acetonitrile, using an organic base such as triethylamine, pyridine or dimethylaniline or an inorganic carbonate such as potassium carbonate or sodium carbonate as a de-hydrogen halide agent at room temperature or under heating if required, with an equi-molar or excess amount of a corresponding carboxylic acid halide, carboxylic acid anhydride or haloformic acid ester and then applying usual post-treatment.

The compound represented by the formula (VI)′ can be obtained through the step (e) by treating compound represented by the formula (IV)′ with an oxidizing agent such as chromium oxide, bromine, silver (I) oxide or a combination of dimethylsulfoxide and anhydrous acetic acid.

The compound represented by the formula (VII)′ can be obtained through the step (f) by reacting a compound shown by the (II)′ in an inert solvent such as benzene, toluene, xylene, diethyl ether, dioxane, tetrahydrofuran, methylene chloride, chloroform, methyl ethyl ketone and acetonitrile, by blowing a gaseous ammonia or adding an aqueous ammonia solution, at room temperature or under heating if required and then applying usual post treatment.

The compounds represented by the general formulae (II)′, (VI)′, (V)′, (VI)′, (VII)′ and (X)′, in a case where $R^3$ is other than a hydrogen atom, are mixtures of compounds having 6-substituent and 7-substituent. A desired compound having the 7-substituent can be obtained from each of the mixtures in an optional step by means of separation such as recrystallization or silica gel column chromatography.

In addition, the compound of the general formula (I) in which both of $R^1$ and $R^2$ are hydrogen atoms can be produced by the following step (Synthesis Route (B)).

Synthesis Route (B)

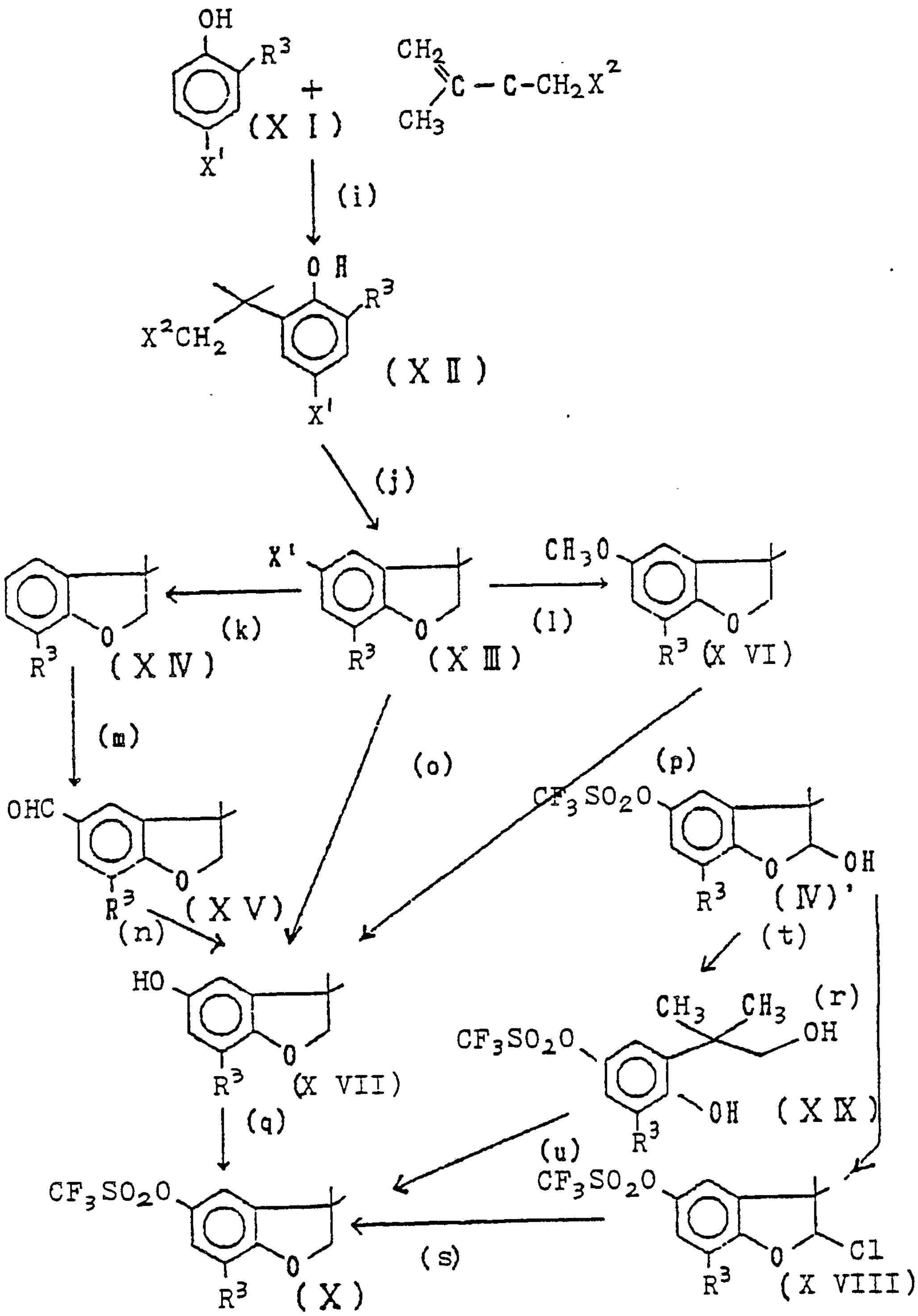

where $R^3$ has the meanings as described above, $X^1$ and $X^2$ represent, independently of each other, a chlorine atome or a bromine atom.

That is, the compound can be produced by a method of reacting 2,3-dihydro-3,3-dimethyl-5-hydroxybenzofuran derivative with or without 7-substituent represented by the general formula (XVII) through the step (q) with trifluoromethane sulfonyl halide or trifluoromethane sulfonic acid anhydride.

The compound represented by the general formula (XVII) can be reacted in an inert solvent such as benzene, toluene, xylene, chlorobenzene, diethyl ether, dioxane, tetrahydrofuran, methylene chloride, chloroform, methyl ethyl ketone and acetone, using an organic base such as triethylamine, pyridine or dimethylaniline or an inorganic carbonate such as powderized potassium carbonate or sodium carbonate as the base, with an equi-molar or slight excess amount of trifluoromethane sulfonyl halide or trifluoromethane

sulfonic acid anhydride. It is also possible to conduct the reaction by using metal sodium or sodium hydride, etc. or using pyridne also as the reaction solvent.

The reaction temperature can be from -50° C to the boiling point of the solvent, but it is advantageous to conduct the reaction at a relatively low temperature region. After the reaction is over, the aimed product can be obtained by applying usual post treatment.

The compound represented by the general formula (XVII) used as the starting material for the reaction can be induced by one of the following three methods using a benzofuran derivative shown by the general formula (XIII) having a chlorine atom or a bromine atom at 5-position.

One of them is a method of dehalogenating a compound represented by the general formula (XIII) by hydrogenolysis through the step (k) into a compound represented by the general formula (XIV), then subjecting to formulation reaction through the step (m) into a compound represented by the general formula (XV) and then further subjected to Derkin reaction into a compound represented by the general formula (XVII). The second method comprises obtaining the compound represented by the general formula (XVII) by converting a compound represented by the general formula (XIII) into a Grignard's reaction through the step (o) and then oxidizing it by oxygen into a compound represented by the general formula (XVII). The last method comprises methoxylating a compound represented by the general formula (XIII) throught the step (1) into a compound represented by the general formula (XVI), then subjecting to ether cleavage in the step (p) to obtain a compound represented by the general formula (XVII).

In the step (k), a compound represented by the general formula (XIII) is reacted in a solvent of water, methanol, ethanol, isopropanol, ethylene glycol, benzene, toluene, acetic acid and ethyl acetate either alone or as a combination of two or more of them, by using a catalyst such as palladium carbon or Raney nickel, and using hydrogen or hydrogen donor which evolves hydrogen gas upon decomposition, e.g., formic acid. The reaction can be conducted under a normal or elevated pressure and the reaction temperature can be from 0° C to the boiling point of the solvent under a normal pressure. The reaction is possible within a temperature range from 0° C to 200° C under an elevated pressure, but it is preferably, conducted within a range from 50 to 150° C. After the reaction is over, the aimed product can be obtained by applying usual post treatment.

In the step (m), a compound represented by the general formula (XIV) is subjected to formylating reaction in a dimethylformamide solvent by using a Vielsmeier reagent prepared from dimethylformamide and phosphorus oxychloride, thionyl chloride or phosgen. The reaction can be conducted at a temperature from 0° C to the boiling point solvent, but it is preferably carried out at a temperature from 50° C to 150° C. After the reaction is over, the aimed product can be obtained by applying usual post treatment.

In the step (n), a compound represented by the general formula (XV) is reacted in a polar solvent such as water, methanol ethanol and propanol, under the presence of a catalytic amount of concentrated sulfuric acid, methane sulfonic acid or trifluoromethane sulfonic acid with an equi-molar or excess amount of hydrogen peroxide.

The reaction temperature can be from 0° C to the boiling point of the solvent but the reaction is preferably carried out around the room temperature. After the reaction is over, the aimed product can be obtained by applying usual post treatment.

In the step (o), a compound represented by the general formula (XIII) is formed into a Grignard's reagent in a solvent such as ether, dioxane or tetrahydrofuran while adding a catalytic amount of iodine if required, and using metal magnesium. Then, oxidative reaction is carried out by blowing an oxygen gas. The reaction can be carried out at a temperature from 0° C to the boiling point of solvent. After the reaction is over, the aimed product can be obtained by applying usual post treatment.

In the step (1), a compound represented by the general formula (XIII) is reacted with sodium methylate in a non-protonic polar solvent such as dimethylformamide, dimethylsulfoxide, N-methyl pyrrolidone or 1,3-dimethyl-2-imidazolidinone by using a transition metal salt such as copper iodide as a catalyst. The reaction may also be conducted by using methanol together with the solvent described above. The reaction can be conducted at a temperature from a room temperature to the boiling point of the solvent but it is preferably conducted at a temperature higher than 80° C. After the reaction is over, the aimed product can be obtained by applying usual post treatment.

In the step (p), well-known dehydrating reagents or methods are employed, i.e., a method of dealkylating a compound represented by the general formula (XVI) in a high boiling solvent such as dimethylformamide, dimethylsulfoxide, N-methylpyrrolidone, 1,3-dimethyl-2-imidazolidine by using sodium thiolate such as sodium ethane thiolate or sodium methane thiolate, or a method of dealkylation in an inert solvent such as chloroform, dichloromethane or dichloroethane by using a Lewis acid such as boron tribromide, boron trichloride or boron trioidide. The reaction can be carried out at a temperature from 0° C to the boiling point of the solvent in both of the methods but it is preferred to carry out the reaction at a

temperature higher than 100° C in the former and near the room temperature in the latter.

The compound represented by the general formula (XIII) can be prepared through the step (j) by reacting a compound represented by the general formula (XII) in one or more of solvents selected from water, methanol, ethanol, tetrahydrofuran, dioxane, diethyl ether, benzene, toluene, xylene, chlorobenzene, methylene chloride, chloroform, methyl ethyl ketone, acetone, N,N-dimethylformamide, N-methylpyrrolidone, 1,3-dimethyl-2-imidazolidinone or dimethylsulfoxide, by adding a base selected from sodium hydroxide, potassium hydroxide, potassium carbonate, sodium carbonate. The reaction can be conducted at a temperature from 0° C to the boiling point of the solvent and, after the reaction is over, the aimed compound represented by the formula (XIII) can be obtained by applying usual post treatment.

The compound represented by the general formula (XII) can be prepared through the step (i) by reacting a phenol derivative represented by the general formula (XI) in a solvent selected from dichloromethane, carbondisulfide and chloroform or without solvent, using an acid selected from concentrated sulfuric acid, phosphoric acid and trifluoromethane sulfonic acid as a catalyst and adding at least an equimolar amount methallyl halide. The reaction temperature can be from -20° C to the boiling point of the solvent and, after the reaction is over, the aimed compound represented by the general formula (XII) can be obtained by applying usual post treatment.

The compound of the present invention represented by the general formula (X) can be obtained by two methods also starting from the general formula (IV)′ des cribed above. One of them is a method of chlorinating a compound represented by the general formula (IV)′ through the step (r) into a compound represented by the general formula (XVIII) and then dehalogenating the same through the step (s) into a compound represented by the general formula (X). In the other of the methods, the compound represented by the general formula (IV)′ is subjected to reduction and ring opening through the step (t) into a compound represented by the general formula (XIX) and then subjected to dehydration and ring closure in the step (u) to obtain a compound represented by the general formula (X).

In the step (r), a compound represented by the general formula (IV)′ is chlorinated in an inert solvent such as benzene, toluene, xylene, diethyl ether, dioxane, tetrahydrofuran, methylene chloride, chloroform, methyl ethyl ketone, acetone or acetonitrile or without such a solvent, using at least an equi-molar amount of thionyl chloride and at least an equi-molar amount of pyridine at a room temperature or under heating if required. After the reaction is over, the aimed compound can be obtained by applying usual post treatment.

In the step (s), a compound represented by the general formula (XVIII) is subjected to dechlorination in an inert solvent such as benzene, toluene, xylene, diethyl ether, dioxane, tetrahydrofuran, methylene chloride, chloroform, methyl ethyl ketone, acetone or acetonitrile or without such a solvent, using an equimolar or slightly excess amount of tributyl tin hydride at room temperatue or under heating if required. After the reaction is over, the aimed product can be obtained by applying usual heat post treatment.

In the step (t), the compound represented by the general formula (IV)′ is subjected to reduction in a mixed solvent comprising a solvent of methanol, ethanol, isopropanol, tetrahydrofuran or dioxane and water, adding an excess sodium hydroxide and sodium borohydride at room temperature or under heating if required. After the reaction is over, the aimed product can be obtained by applying usual treatment.

In the step (u), the compound represented by the general formula (XIX) is subjected to intra-molecular ring closure without solvent or in an inert solvent such as benzene, toluene, xylene, chlorobenzene, dioxane, tetrahydrofuran, methylene chloride, chloroform, methyl ethyl ketone, acetone or N,N-dimethylformamide, by using a Vilsmeier reagent comprising the combination of thionyl chloride and N,N-dimethylformamide, etc. or a dehydrating agent such as phosphorus pentoxide, dicyclohexyl carbodiimide, sulfuric acid or phosphoric acid at room temperature or under heating if required. After the reaction is over, the aimed product can be obtained by applying usual post treatment.

The compound represented by the general formula (XVIII) or (XIX) derived from (IV)′, in a case where $R^2$ is other than hydrogen atom, is a mixture of those having 6-substituent and those having 7-substituent. A desired 7-substituted compound can be obtained from each of the mixtures by separation means such as recrystallization or silica gel column chromatography at any stage.

The compound represented by the general formula (I) according to the present invention is extremely safe to the rice plant in paddy field and, on the other hand, shows strong herbicidal effect against annual weeds such as Echinochloa crus-galli, Cyperus difformis, Monochroria vaginalis and Ratala indica, as well as against perennial weeds such as Scirpus juncoides, Cyperus serotinus, Eleocharis kuroguwai, Eleocharis acicularis and Sagittaria pygmaea, thus have excellent performance as a herbicide for the rice plant in paddy field. Further, by means of preemergence treatment or postemergence treatment, it is also effective against Digitaria sanguinalis, Stellaria media, Polygonum lapathifolium, Amaranthus lividus, Cyperus iria, Portulaca oleracea, Senecio vulgaris, Chenopodium album, Cyperus rotundus, Sagina japonica, Galium spurium, Alopecurus aequalis, Pea annua, Capsella bursa-pastoris and Setaria viridis. also effective as a

herbicide in upland and other non-agricultural land.

Referring to the compound according to the present invention represented by the general formula (I), in which $R^3$ is hydrogen atom, although USP-3,689,507, USP-3,937,702, USP-4,051,154, USP-4,120,871 and USP-4,162,154 disclose a chloromethane sulfonate and 3-chloropropane sulfonate forms which are similar to the compound of the present invention in that they are halogen-substituted alkyl sulfonates, the trifluoromethane sulfonate form of the compound according to the present invention is not disclosed at all. For the known chloro-substituted alkyl sulfonate forms, superior herbicidal activities and other points can not be found as compared with corresponding non-substituted alkyl sulfonate forms in view of the test examples in the publications. On the other hand, the trifluoromethane sulfonate derivatives as the compound according to the present invention, as detailed in the subsequent test examples, show remarkably improved herbicidal activity as compared with the corresponding not-substituted alkyl sulfonate form and, at the same time, show improved safety to the rice plant in paddy field outstandingly higher than similar known compounds. Thus, the compounds according to the present invention, although belonging to the same halogen-substituted alkyl sulfonate forms, are quite different in the nature from the known compounds and can not be anticipated with ease even by those skilled in the art.

In the compounds according to the present invention represented by the general formula (I) in which $R^3$ is methyl group or halogen atom, different from the known compounds as disclosed in USP-3,689,507 and USP-3,937,702, presence or absence of the methyl group or halogen atom at 6- or 7-position has a great influence on the herbicidal activity. That is, although the above-mentioned patent publications shows examples of compounds having alkyl group or halogen atom on 6-, 4,6- and 6,7-positions on the benzene ring in the benzofurane ring, it can not be recognized that the compounds having these substituents show excellent herbicidal effect as compared with the non-substituted compounds in view of the test examples therein.

On the contrary, in the compounds which is substituted at the 5-position with a trifluoromethanesulfonyloxy group, the herbicidal activity is remarkably reduced in a compound having a methyl group or halogen atom on the 6-position as compared with a compound having no such substituent. On the other hand, when the methyl group or halogen atom is introduced to the 7-position as in the present invention, the herbicidal effect is outstandingly improved and, further, safety to the paddy field rice plant is also improved. From the fact as described above, combination of the trifluoromethanesulfonyloxy group at 5-position and the methyl group or halogen atom at 7-position is extremely important in view of the herbicidal activity and the safety.

The compound according to the present invention represented by the general formula (I) can be used as it is to plants to be treated but it is generally used in admixture with an inert liquid or solid to be prepared into a formulation, for example, dust, granule, wettable powder, emulsifiable concentrate, flowable formulation, etc. employed usually. Further, adjuvants may also be added if required in view of formulation.

As a carrier, either solid or liquid carrier may be used so long as it is usually used for agricultural and horticultural chemicals, with no restriction to particular materials. As the solid carrier, there can be mentioned, for example, mineral powder such as clay, talc, bentonite, calcium carbonate, diatomaceous earth or white carbon, vegetable powder such as soybean powder or starch, polymeric compound such as petroleum resin, polyvinyl alcohol or polyalkylene glycol, urea, wax, etc. Further, as the liquid carrier, there can be mentioned various kinds of oils and various kinds of organic solvent, water, etc.

As the adjuvant, surface active agent, binder, stabilizer, etc. usually employed for agricultural and horticultural chemicals may be used alone or in combination as required. Furthermore, industrial bacteriocide or anti-bacteria or antifungus agent may also be added with an aim of controlling bacteria and fungi.

As the surface active agent, nonionic, anionic, cationic and amphoteric agents may be used. Preferred nonionic surface active agents are polyethyleneoxide alkylphenyl ether, polyoxyethylene alkyl ether, polyoxyethylene alkylnaphthalene ether and polyethylene glycol fatty acid ester as polymers of alkylphenol, higher alcohol, alkylnaphthol, or higher fatty acid and ethylene oxide, or polyoxyethylene - polyoxypropylene block polymer polymerized from ethylene oxide and propylene oxide. Preferred anionic surface active agents are salts of alkylphenol sulfate, alkylphenol phosphate, alkylnaphthol sulfate, alkylnaphthol phosphate, higher alcohol sulfate or higher alcohol phosphate which are salts of alkylphenol, alkylnaphthol or higher alcohol with sulfuric acid ester or phosphoric acid ester, alkylbenzene sulfonate, alkylnaphthalene sulfonate, as well as salts of higher fatty acids such as fatty acid soaps.

The content of the compound represented by the general formula (I) in the herbicide according to the present invention, while varying depending on the formulations, is usually from 0.5 to 20 % by weight for the dust, from 10 to 60 % by weight for the wettable powder, from 0.5 to 30% by weight for the granule, from 1 to 50 % by weight for the emulsifiable concentrate, from 10 to 50 % by weight for the flowable

formulation and from 20 to 60% by weight for the dry flowable formulation. Preferably, it is from 1 to 5 % by weight for the dust, from 20 to 40 % by weight for the wettable powder, from 1 to 5 % by weight for the granule, from 10 to 20 % by weight for the emulsifiable concentrate, from 20 to 30 % by weight for the flowable formulation and from 20 to 40 % by weight for the dry flowable formulation.

The content of the adjuvant is from 0 to 80 % by weight, and the content of the carrier is the balance of the content of the effective ingredient compound and the adjuvant subtrated from 100 % by weight.

The herbicide according to the present invention is effective in all of treating methods such as flooded soil treatment, soil treatment, soil incorporation treatment, spraying treatment to stalks and leaves, etc. The application amount can be in a wide range from 0.01 kg to 10 kg/ha and, generally, it is used preferably within a range from 0.1 kg to 5 kg/ha.

The herbicide according to the present invention can be used in admixture with other agricultural chemicals such as one or more of other herbicides, insecticides, plant growth controlling agent, soil improver or fertilizing material, as well as can be used in the form of a formulation mixed therewith. Depending on the case, a synergistic effect can also be expected. In this case, it is particularly advantageous to be used in admixture with other herbicides.

As other herbicides, there can be mentioned, for example, organic herbicides such as phenoxy acetate herbicide, benzoic acid herbicide, chlorinated carboxylic acid herbicide, carbamate herbicide, urea herbicide, sulfonylurea herbicide, acid amide herbicide, heterocyclic herbicide (triazine herbicide, diazine herbicide, etc.), phenolic herbicide, diphenyl ether herbicide, dipyrimidinium herbicide, dinitroaniline herbicide, organic phosphate herbicide, phosphorus-containing amino acid herbicide, imidazolidinone herbicide, pyridine herbicide, quinoline herbicide, sulfon amide herbicide, cyclohexanone herbicide and like other organic herbicides, as well as inorganic herbicides.

The present invention is to be described in more details referring to examples.

## EXAMPLE

### Example 1

Preparation of 2,3-dihydro-3,3-dimethyl-2-morpholino-5-trifluoromethanesulfonyloxy benzofuran (Compound No. 2)

2,3-dihydro-3,3-dimethyl-5-hydroxy-2-morpholinobenzofuran was dissolved by 5.0 g into 50 ml of acetonitrile, to which 4 ml of triethylamine was added. Then, 2,2 ml of trifluoromethane sulfonyl chloride was gradually dropped under ice cooling and, subsequently, they were stirred at 30 °C for 4 hours to complete the reaction. After allowing them to cool, insoluble matters were removed by filtration, the solvent was distilled off under a reduced pressure and the resultant residue was dissolved in ethyl acetate, washed with water and dried. Then, after distilling off the solvent, the residue was purified on silica gel chromatograph (eluent; N-hexane : ethyl acetate = 9:1), to obtain 6.4 g (84 % yield) of oily 2,3-dihydro-3,3-dimethyl-2-morpholino-5-trifluoromethanesulfonyloxy benzofuran.

### Example 2

Preparation of 2,3-dihydro-3,3-dimethyl-2-hydroxy-5-trifluoromethanesulfonyloxy benzofuran (Compound No. 4)

2,3-dihydro-3,3-dimethyl-2-morpholino-5-trifluoromethanesulfonyloxy benzofuran was suspended by 6.0 g into 4.5 ml of 35% hydrochloric acid and 11 ml of water, and reacted, while stirring violently, at 90 °C for 10 min and then allowed to cool. The ether layer was washed with water and dried and then solvent was distilled off. The residue was purified on silica gel chromatograph (eluent; n-hexane : ethyl acetate = 8 : 2), to obtain 4.6 g (94% yield) of oily 2,3-dihydro-3,3-dimethyl-2-hydroxy-5-trifluoromethanesulfonyloxy benzofuran.

Example 3

Preparation of 2,3-dihydro-3,3-dimethyl-2-(3,4-dichlorobenzoyloxy)-5-trifluoromethanesulfonyloxy benzofuran (Compound No. 8)

2,3-dihydro-3,3-dimethyl-2-hydroxy-5-trifluoromethanesulfonyloxy benzofuran obtained in Example 2 was dissolved by 1.2 g into 50 ml of dimethyl ether, to which 0.7 ml of triethylamine was added. 0.8 g of 3,4-dichlorobenzoyl chloride was gradually dropped under ice cooling. After stirring them at a temperature of lower than 10 °C for 3 hours, the reaction solution was poured into water. The ether layer obtained by separation was further washed with water and dried and then the solvent was distilled off. The resultant crude products were purified on silica gel chromatograph (eluent; n-hexane : ethyl acetate = 19 : 1), to obtain 1.4 g (78 % yield) of oily 2,3-dihydro-3,3-dimethyl-2-(3,4-dichlorobenzoyloxy)-5-trifluoromethanesulfonyloxy benzofuran.

Example 4

Preparation of 2,3-dihydro-3,3-dimethyl-2-oxo-5-trifluoromethanesulfonyloxy benzofuran (Compound No. 9)

2,3-dihydro-3,3-dimethyl-2-hydroxy-5-trifluoromethanesulfonyloxy benzofuran obtained in Example 2 was added by 3.1 g to a mixed solution of 8 ml of dimethyl sulfoxide and 5 ml of anhydrous acetic acid. After leaving the solution at a room temperature for 3 days, it was poured into iced water and oily products were extracted with ether. After water washing and drying the ether layer, the solvent was distilled off. The residue was purified on silica gel chromatograph (eluent; n-hexane : ethyl acetate = 8 : 2), to obtain 3.0 g of 2,3-dihydro-3,3-dimethyl-2-oxo-5-trifluoromethanesulfonyloxy benzofuran. (96% yield)

Example 5

Preparation of 2,3-dihydro-3,3-dimethyl-5-trifluoromethanesulfonyloxy benzofuran (Compound No. 1)

To a 50 ml solution of tetrahydrofuran in which 3.3 g of 2,3-dihydro-3,3-dimethyl-5-hydroxybenzofuran and 2.1 g of triethylamine were dissolved, 3.4 g of trifluoromethansulfonyl chloride was added by dropping while maintaining the temperature to lower than 10 °C. After stirring at a room temperature for two hours, the reaction solution was poured into water and extracted with ethyl acetate. The organic layer was dried over anhydrous sodium sulfate and then the solvent was distilled off. The residue was purified on silica gel chromatograph (eluent; n-hexane : ethyl acetate = 10 : 1), to obtain 5.6 g of 2,3-dihydro-3,3-dimethyl-5-trifluoromethanesulfonyloxy benzofuran.

Example 6

Preparation of 2-amino-2,3-dihydro-3,3-dimethyl-5-trifluoromethanesulfonyloxy benzofuran (Compound No. 14)

2,3-dihydro-3,3-dimethyl-2-morpholino-5-trifluoromethanesulfonyloxy benzofuran obtained in Example 1 was dissolved by 3.8 g into 10 ml of acetone, to which 50 ml of an aqueous 28% ammonia solution was added and they were stirred under heating for six hours while refluxing by using a condenser cooled with dry ice and acetone to complete the reaction. After allowing the reaction solution to cool, the oily product was extracted with addition of ethyl acetate, the organic layer was washed with water and dried and then the solvent was distilled off under a reduced pressure. The resultant residue was purified on silica gel chromatograph (eluent ; n-hexane : ethyl acetate = 4 : 1), to obtain 0.9 g (32% yield) of 2-amino-2,3-dihydro-3,3-dimethyl-5-trifluoromethanesulfonyloxy benzofuran.

The same procedures as those in Examples 1 and 2 described later were applied except for using, as the starting material, 2,3-dihydro-5-hydroxy-2-morpholino-3,3,6-trimethylbenzofuran obtained at the same time with 2,3-dihydro-5-hydroxy-2-morpholino-3,3,7-trimethylbenzofuran obtained in Reference Example 1, and separated by silica gel chromatography, to obtain 2,3-dihydro-2-hydroxy-5-trifluoromethanesulfonyloxy-3,3,6-trimethylbenzofuran.

(NMR Spectrum)

($CHCl_3$) $\delta$ : 1.25 (3H, s), 1.30 (3H, s), 2.28 (3H, s), 3.15 (1H, d, J=4.5Hz), 5.55 (1H, d, J=4.5Hz, 6.68 (1H, s), 6.90 (1H, s)

Example 7

Preparation of 2,3-dihydro-2-morpholino-5-trifluoromethanesulfonyloxy-3,3,7-trimethylbenzofuran (Compound No. 15)

2,3-dihydro-5-hydroxy-2-mopholino-3,3,7-trimethylbenzofuran obtained in Reference Example 1 was dissolved by 7.0 g into 60 ml of acetonitrile, to which 5 ml of triethylamine was added. 3.1 ml of trifluoromethanesulfonyl chloride was gradually dropped under ice cooling and, subsequently, they were stirred at 30 °C for 4 hours to complete the reaction. After allowing the reaction solution to cool, insoluble matters were removed by filtration and the solvent was distilled off under a reduced pressure. After dissolving the resultant residue against into ethyl acetate, it was washed with water and dried and then the solvent was distilled off under a reduced pressure, to obtain oily crude products. The crude products were purified on silica gel chromatography (eluent; n-hexane : ethyl acetate = 9 : 1), to obtain 8.4 g (80% yield) of 2,3-dihydro-2-morpholino-5-trifluoromethane sulfonyloxy-3,3,7-trimethylbenzofuran.

Example 8

Preparation of 2,3-dihydro-2-hydroxy-5-trifluoromethanesulfonyloxy-3,3,7-trimethylbenzofuran (Compound No. 16)

2,3-dihydro-2-mopholino-5-trifluoromethanesulfonyloxy-3,3,7-trimethylbenzofuran was suspended by 5.5 g into 4.5 ml of 35% hydrochloric acid and 11 ml of water and reacted while violently stirring at 90 °C for 10 min. After the reaction was over, it was allowed to cool and oily products were extracted with ether, washed with water and then dried. Then, the residue obtained by distilling off the solvent was purified on silica gel chromatography (eluent; n-hexane : ethyl acetate = 8 : 2), to obtain 4.1 g (91% yield) of 2,3-dihydro-2-hydroxy-5-trifluoromethanesulfonyloxy-3,3,7-trimethylbenzofuran.

Example 9

Preparation of 2-benzoyloxy-2,3-dihydro-5-trifluoromethanesulfonyloxy-3,3,7-trimethylbenzofuran (Compound No. 19)

2,3-dihydro-2-hydroxy-5-trifluoromethanesulfonyloxy-3,3,7-trimethylbenzofuran obtained in Example 8 was dissolved by 2.0 g into 50 ml of dimethyl ether, to which 1,3 ml of triethylamine was added, and then, 0.9 g of benzoyl chloride was gradually dropped under ice cooling. Subsequently, after stirring for 3 hours at a temperature lower than 10 °C, the reaction solution was poured into water. After further water washing and drying the ether layer obtained by separation, the solvent was distilled off to obtain oily crude products. The resultant crude products were purified on silica gel chromatography (eluent; n-hexane : ethyl acetate = 19 : 1), to obtain 2.0 g (76% yield) of 1-benzoyloxy-2,3-dihydro-5-trifluoromethanesulfonyloxy-3,3,7-trimethylbenzofuran.

Example 10

Reference Example 1

Preparation of 2,3-dihydro-5-hydroxy-2-morpholino-3,3,7-trimethylbenzofuran

(1) Preparation of enamine

To a 50 ml of toluene solution containing 15.8 g of isobutyl aldehyde, 9.6 g of morpholine was added under stirring. The mixture was refluxed under heating for three hours while continuously separating and removing resultant water to prepare enamine.

(2) Preparation of 2,3-dihydro-5-hydroxy-2-morpholino-3,3,7-trimethylbenzofuran

12.2 g of 2,5-toluquinone was suspended into 15 ml of toluene, to which the entire amount of the enamine solution obtained by the method as described above was added gradually and then mixed while heating under reflux for 30 min. After sludging the residue obtained by distilling off the toluene from the reaction solution with a small amount of ether and hexane, solid matters were filtered, then washed with a small amount of toluene and dried to obtain a mixture of the aimed product and 2,3-dihydro-5-hydroxy-2-morpholino-3,3,6-trimethylbenzofuran as the positional isomer thereof. It was purified twice on silica gel chromatography (eluent ; n-hexane : ethyl acetate = 8 : 2 and chloroform : methanol = 40 : 1), to obtain 11.9 g of the aimed 2,3-dihydro-5-hydroxy-2-morpholino-3,3,7-trimethylbenzofuran (45% yield).

Reference Example 2

Preparation of 5-ethanesulfonyloxy-2,3-dihydro-3,3,7-trimethylbenzofuran (Comparative Compound E)

5-ethanesulfonyloxy-2,3-dihydro-3,3,7-trimethylbenzofuran was prepared in the same procedures as those in Examples 1, 2 and 5 described later except for using 2,3-dihydro-2-morpholino-5-hydroxy-3,3,7-trimethylbenzofuran obtained in Reference Example 1 as the starting material and using ethane sulfonyl chloride instead of trifluoromethane sulfonyl chloride.
(NMR Spectrum)
($CDCl_3$) $\delta$ : 1.33 (6H, s), 1.53 (3H, t), 2.20 (2H, s), 3.24 (2H, q), 4.25 (2H, s), 6.88 (2H, bs)

Reference Example 3

Preparation of 5-(3-chloropropane sulfonyloxy)-2,3-dihydro-3,3,7-trimethylbenzofuran (Comparative Compound F)

5-(3-chloropropane sulfonyloxy)-2,3-dihydro-3,3,7-trimethylbenzofuran was prepared in the same procedures as those in Examples 1, 2 and 5 described later except for using 2,3-dihydro-2-morpholino-5-hydroxy-3,3,7-trimethylbenzofuran obtained in Reference Example 1 as the starting material and using 3-chloropropane sulfonyl chloride instead of trifluoromethane sulfonyl chloride.
(NMR Spectrum)
($CDCl_3$) $\delta$ : 1.33 (6H, s), 2.21 (3H, t), 2.45 (2H, m), 3.42 (2H, 6, J=7.2Hz), 3.77 (2H, t, J=6.2Hz), 4.27 (2H, s), 6.85 (2H, s)

Reference Example 4

Preparation of 2,3-dihydro-2-hydroxy-5-trifluoromethanesulfonyloxy-3,3,6-trimethylbenzofuran (Comparative Compound G)

Preparation of 2,3-dihydro-2-oxo-5-trifluoromethanesulfonyloxy-3,3,7-trimethylbenzofuran (Compound No. 17)

2,3-dihydro-2-hydroxy-5-trifluoromethanesulfonyloxy-3,3,7-trimethylbenzofuran was added to a mixed solvent of 4 ml of dimethylsulfoxide and 2.8 ml of anhydrous acetic acid and left at a room temperature for three days. The reaction solution was poured into iced water, separated oily products were extracted with ether and the ether layer was washed with water and dried and then the solvent was distilled off. The residue was purified on silica gel chromatography (eluent; n-hexane : ethyl acetate = 9 : 1), to obtain 1.37g (92% yield) of 2,3-dihydro-2-oxo-5-trifluoromethanesulfonyloxy-3,3,7-trimethylbenzofuran.

Example 11

Preparation of 2,3-dihydro-5-trifluoromethanesulfonyloxy-3,3,7-trimethylbenzofuran (Compound No. 20)

(1) Preparation of 2-chloro-2,3-dihydro-5-trifluoromethanesulfonyloxy-3,3,7-trimethylbenzofuran

2,3-dihydro-2-hydroxy-5-trifluoromethanesulfonyloxy-3,3,7-trimethylbenzofuran was dissolved by 1.6 g into 10 ml of dichloromethane, to which 0.5 ml of pyridine was added and, under ice cooling, 0.4 ml of thionyl chloride was gradually charged by dropping. Subsequently, after stirring at a temperature lower than 20 °C for 3 hours, the reaction solution was poured into water. After washing the dichloromethane layer obtained by separation with aqueous sodium hydrogen carbonate solution and then with water and drying, the solvent wave distilled off. The resultant crude products were purified on silica gel chromatography (eluent; n-hexane : ethyl acetate = 19 : 1), to obtain 1.4 g (82% yield) of oily 2-chloro-2,3-dihydro-5-trifluoromethanesulfonyloxy-3,3,7-trimethylbenzofuran

(2) Preparation of 2,3-dihydro-5-trifluoromethanesulfonyloxy-3,3,7-trimethylbenzofuran

After dissolving 1.0 g of 2-chloro-2,3-dihydro-5-trifluoromethanesulfonyloxy-3,3,7-trimethylbenzofuran into 8 ml of xylene, 0.1 g of 2,2-azobisisobutyronitrile and 0.8 ml of tributyl tin hydride were added and refluxed in a nitrogen atmosphere for 2 hours to complete the reaction. After discharging the reaction solution into iced water, the xylene layer was washed with water and dried and then the solvent was distilled off under a reduced pressure. The residue was purified on silica gel chromataography (eluent; n-hexane : ethyl acetate = 40 : 1), to obtain 0.86 g (96% yield) of 2,3-dihydro-5-trifluoromethanesulfonyoxy-3,3,7-trimethylbenzofuran.

Example 12

Preparation of 2,3-dihydro-3,3-dimethyl-7-fluoro-5-trifluoromethanesulfonyloxy benzofuran (Compound No. 9)

(1) Preparation of 2-(2-chloro-1,1-dimethylethyl)-4-bromo-6-fluorophenol

4-bromo-2-fluorophenol was dissolved by 50 g into 100 ml of methylene chloride, to which 26 g of concentrated sulfuric acid was added. Then, 59.8 g of β-methallyl chloride was charged by dropping for about one hour while maintaining the internal temperature to lower than 5 °C. Then, they were stirred at room temperature for 2 hours and then poured into 500 ml of water.

Separated oily products were extracted with a solvent mixture comprising 200 ml of ethylene acetate and 200 ml of n-hexane, washed with water and dried over anhydrous sodium sulfate and then the solvent was distilled off under a reduced pressure, to obtain 57.3 g of oily crude products. The crude products were purified on silica gel column chromatography to obtain 12.7 g (17.1% yield) of aimed 2-(2-chloro-1,1-dimethylethyl)-4-bromo-6-fluorophenol.

(2) Preparation of 5-bromo-2,3-dihydro-3,3-dimethyl-7-fluorobenzofuran

After dissolving 8.45 g of 2-(2-chloro-1,1-dimethylethyl)-4-bromo-6-fluorophenol obtained in (1) above into 15 ml of tetrahydrofuran, 50 ml of an aqueous 20% sodium hydroxide solution was added and stirred at room temperature for 3 hours to complete the reaction. After acidifying the reaction solution using concentrated hydrochloric acid, it was extracted with 300 ml of n-hexane and the n-hexane layer was washed with water. It was dried over anhydrous sodium sulfate and the solvent was distilled off under a reduced pressure to obtain crude products.

The crude products were purified on silica gel chromatography (n-hexane), to obtain 3.16 g (43.0% yield) of aimed 5-bromo-2,3-dihydro-3,3-dimethyl-7-fluorobenzofuran.

(3) Preparation of 2,3-dihydro-3,3-dimethyl-7-fluoro-5-hydroxybenzofuran

0.3 g (12.2 mmol) of magnesium and a catalytic amount of iodine were added into 10 ml of tetrahydrofuran and refluxed under heating, into which 10 ml solution of tetrahydrofuran containing 3 g of 5-brom-2,3-dihydro-3,3-dimethyl-7-fluorobenzofuran obtained in (2) above was charged by dropping for about 30 min. Then, refluxing was continued under heating for one hour to prepare a Grignard's reagent.

Then, while cooling the reaction solution to a temperature lower than 30° C, an oxygen gas was blown and after the evolution of heat was no more recognized, the oxygen gas was blown under reflux while heating for 2 hours to complete the reaction. After cooling, the reaction solution was poured into about 100 ml of water and acidified with a concentrated hydrochloric acid, and then oily products were extracted using 100 ml of ethyl acetate. After washing the organic layer with an saturated aqueous solution of sodium chloride, it was dried over anhydrous sodium sulfate and the solvent was distilled off under a reduced pressure to obtain crude products.

The crude products were purified on silica gel column chromatography to obtain 0.58 g (26.0% yield) of aimed 2,3-dihydro-3,3-dimethyl-7-fluoro-5-hydroxybenzofuran.

(4) Preparation of 2,3-dihydro-3,3-dimethyl-7-fluoro-5-trifluoromethanesulfonyloxy benzofuran

0.55 g of 2,3-dihydro-3,3-dimethyl-7-fluoro-5-hydroxybenzofuran obtained in (3) above and 0.5 g of triethylamine were dissolved into 10 ml of tetrahydrofuran, to which 0.84 g of trifluoromethane sulfonyl chloride was charged by dropping, while cooling to a temperature lower than 5° C for about 10 min, and then stirred at room temperature for 2 hours to complete the reaction.

The reaction solution was poured into about 100 ml of water, an oily portion was extracted with 100 ml of ethyl acetate. After washing with an aqueous saturated solution of sodium chloride, it was dried over anhydrous sodium sulfate and then the solvent was distilled off under a reduced pressure, to obtain 0.84 g (89.5% yield) of aimed 2,3-dihydro-3,3-dimethyl-7-fluoro-5-trifluoromethanesulfonyloxy benzofuran.

Melting point and NMR spectral data are shown for typical 2,3-dihydrobenzofuran derivatives represented by the general formula (I) according to the present invention in Table 1.

Table 1

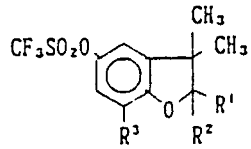

(I)

| Compound No. | $R^1$ | $R^2$ | $R^3$ | mp (℃) | NMR Spectra |
|---|---|---|---|---|---|
| 1 | H | H | H | oily | ($CCl_4$) δ: 1.37(6H,s),4.23(2H,s),6.6~6.8 (1H,m),6.9~7.1(2H,m) |
| 2 | 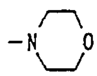 | ″ | ″ | 56 ~ 58 | ($CDCl_3$) δ : 1.27(3H,s),1.38(3H,s),2.46~2.84 (4H,m),3.62(4H,t,J=5.0Hz),4.89 (1H,s),6.76 ~7.14(3H,m) |
| 3 | $-OC_2H_5$ | ″ | ″ | oily | ($CDCl_3$) δ : 1.1 ~1.4(3H,m),1.27(3H,s),1.30 (3H,s),3.5~4.1(2H,m),5.28(1H,s) 6.7 ~7.4(3H,m) |
| 4 | -OH | ″ | ″ | ″ | ($CDCl_3$) δ : 1.29(3H,s),1.35(3H,s),3.35(1H,s) 5.61(1H,s),6.80~7.06(3H,m) |
| 5 | $-O\overset{O}{\overset{\|}{C}}CH_3$ | ″ | ″ | ″ | ($CDCl_3$) δ: 1.34(3H,s),1.38(3H,s),2.11(3H,s) 6.42(1H,s),6.88~7.06(3H,m) |
| 6 | $-O\overset{O}{\overset{\|}{C}}OC_4H_9$ | ″ | ″ | ″ | ($CDCl_3$) δ : 0.91~0.98(3H,m),1.35 ~1.46 (8H,m),1.61 ~1.75(2H,m), 4.18~4.27(2H,m),6.28(1H,s), 6.89~7.29(3H,m) |
| 7 | 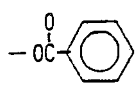 | H | ″ | ″ | ($CDCl_3$) δ: 1.42(3H,s),1.51(3H,s),6.67(1H,s) 6.90~7.11(3H,m),7.41~7.61 (3H,m), 8.00~8.04(2H,m) |
| 8 | 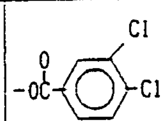 | ″ | ″ | ″ | ($CDCl_3$) δ: 1.42(3H,s),1.50(3H,s),6.65(1H,s) 6.90~7.13(3H,m),7.46~8.16 (3H,m) |
| 9 | = 0 | | ″ | ″ | ($CDCl_3$) δ: 1.54(6H,s),7.15(3H,m), |
| 10 | 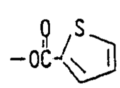 | H | ″ | ″ | ($CDCl_3$) δ: 1.40(3H,s),1.49(3H,s),6.60(1H,s) 6.90~6.94(1H,m),7.01~7.13 (3H,m),7.59 ~7.62(1H,m), 7.82~7.84(1H,m) |

Table 1 (Continued)

| Compound No. | $R^1$ | $R^2$ | $R^3$ | mp (℃) | NMR Spectra |
|---|---|---|---|---|---|
| 11 | 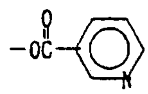 | H | H | oily | ($CDCl_3$) δ: 1.44(3H,s),1.53(3H,s),6.68(1H,s) 6.91~6.96(1H,m),7.08~7.13 (2H,s),7.38 ~7.43(1H,m), 8.27~8.31(1H,m),8.27 ~8.31 (1H,m),8.79 ~8.82(1H,m) |
| 12 | 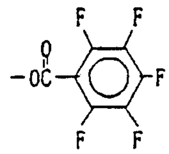 | 〃 | 〃 | 〃 | ($CDCl_3$) δ: 1.41(3H,s),1.47(3H,s),6.63(1H,s) 6.89~7.12(3H,m) |
| 13 | 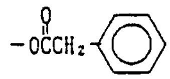 | 〃 | 〃 | 〃 | ($CDCl_3$) δ: 1.26(3H,s),1.31(3H,s),3.66(2H,s) 6.41(1H,s),6.90~7.31(8H,m) |
| 14 | $-NH_2$ | 〃 | 〃 | 134 ~ 136 | ($CDCl_3$) δ: 1.18(3H,s),1.36(3H,s),2.25(2H,s) 5.19(1H,s),6.72(1H,d,J=9Hz), 6.9 ~7.1(2H,m) |
| 15 | 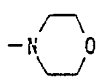 | 〃 | $-CH_3$ | oily | ($CDCl_3$) δ: 1.26(3H,s),1.37(3H,s),2.24(3H,s) 2.46~2.59(2H,m),2.64~2.72 (2H,m),3.58 ~ 3.64(4H,m),4.90 (1H,s),6.75(1H,d,J=2.5Hz),6.85 (1H,d,J=2.5Hz) |
| 16 | −OH | 〃 | 〃 | 〃 | ($CDCl_3$) δ: 1.26(3H,s),1.33(3H,s),2.24(3H,s) 3.14(1H,d,J=5.0Hz),5.60 (1H,d,J=5.0Hz),6.81 (1H,d,J=2.5Hz),6.88(d,J=2.5Hz) |
| 17 | =O | | 〃 | 〃 | ($CDCl_3$) δ: 1.55(6H,s),2.41(3H,s),6.98 (1H,d,J=2.5Hz),7.02 (1H,d,J=2.5Hz) |
| 18 | $-O\overset{O}{\overset{\|}{C}}CH_3$ | H | 〃 | 58~63 | ($CDCl_3$) δ: 1.32(3H,s),1.35(3H,s),2.11(3H,s) 2.25(3H,s),6.44(1H,s),6.84 (1H,d,J=2.5Hz),6.91 (1H,d,J=2.5Hz) |
| 19 | 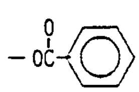 | 〃 | 〃 | 62~65 | ($CDCl_3$) δ: 1.40(3H,s),1.48(3H,s),2.25(3H,s) 6.69(1H,s),6.89(1H,d,J=2.5Hz), 6.92(1H,d,J=2.5Hz),7.42~7.48 (2H,m),7.56 ~7.62(1H,m), 8.01~8.05(2H,m) |
| 20 | −H | 〃 | 〃 | oily | ($CDCl_3$) δ: 1.34(6H,s),2.22(3H,s),4.29(2H,s) 6.81(1H,d,J=2.5Hz),6.85 (1H,d,J=2.5Hz) |

Table (Continued)

| Compound No. | $R^1$ | $R^2$ | $R^3$ | mp (℃) | NMR Spectra |
|---|---|---|---|---|---|
| 21 | 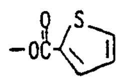 | -H | $-CH_3$ | 89~91 | ($CDCl_3$) δ: 1.38(3H,s),1.46(3H,s),2.26(3H,s) 6.62(1H,s),6.87(1H,d,J=2.5Hz), 6.92(1H,d,J=2.5Hz),7.08~7.13 (1H,m),7.60 ~7.63(1H,m), 7.82~7.85(1H,m) |
| 22 | $-O\overset{O}{\overset{\|}{C}}CHCl_2$ | 〃 | 〃 | oily | ($CDCl_3$) δ: 1.37(3H,s),1.43(3H,s),2.26(3H,s) 5.95(1H,s),6.48(1H,s),6.87 (1H,d,J=2.5Hz),6.94 (1H,d,J=2.5Hz) |
| 23 | -H | 〃 | -F | 〃 | ($CDCl_3$) δ: 1.37(6H,s),4.39(2H,s),6.82 (1H,d,J=2.5Hz),6.89 (1H,dd,J=9.9Hz,J=2.5Hz) |
| 24 | 〃 | 〃 | -Cl | 〃 | ($CDCl_3$) δ: 1.38(6H,s),4.40(2H,s),6.90 (1H,d,J=2.5Hz),7.09 (1H,d,J=2.5Hz) |
| 25 | 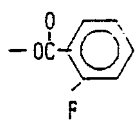 | 〃 | $-CH_3$ | 〃 | ($CDCl_3$) δ: 1.40(3H,s),1.48(3H,s),2.25(3H,s) 6.68(1H,s),6.88(1H,d,J=2.5Hz), 6.91(1H,d,J=2.5Hz),7.09~7.26 (2H,m),7.51~7.59(1H,m), 7.91~7.97(1H,m) |
| 26 | 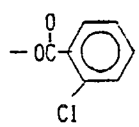 | 〃 | 〃 | 〃 | ($CDCl_3$) δ: 1.40(3H,s),1.46(3H,s),2.26(3H,s) 6.69(1H,s),6.87(1H,d,J=2.5Hz), 6.92(1H,d,J=2.5Hz),7.26~7.34 (1H,m),7.40~7.48(2H,m), 7.80~7.84(1H,m) |
| 27 | 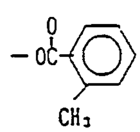 | 〃 | 〃 | 〃 | ($CDCl_3$) δ: 1.40(3H,s),1.46(3H,s),2.26(3H,s) 2.61(3H,s),6.68(1H,s),6.88 (1H,d,J=2.5Hz),6.92 (1H,d,J=2.5Hz),7.19~7.45(3H,m) 7.86~7.89(1H,m) |
| 28 | 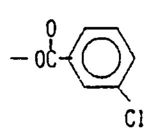 | 〃 | 〃 | 〃 | ($CDCl_3$) δ: 1.40(3H,s),1.47(3H,s),2.26(3H,s) 6.67(1H,s),6.89(1H,d,J=2.5Hz), 6.93(1H,d,J=2.5Hz),7.36~7.42 (1H,m),7.54~7.58(1H,m) 7.90~7.99(2H,m) |
| 29 | 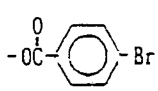 | 〃 | 〃 | 94 ~ 96 | ($CDCl_3$) δ: 1.40(3H,s),1.46(3H,s),2.25(3H,s) 6.67(1H,s),6.88(1H,d,J=2.5Hz), 6.93(1H,d,J=2.5Hz),7.55~7.61 (2H,m),7.86 ~7.91(2H,m) |

Table 1 (Continued)

| Compound No. | $R^1$ | $R^2$ | $R^3$ | mp (℃) | NMR Spectra |
|---|---|---|---|---|---|
| 30 | 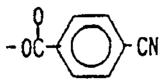 | H | $CH_3$ | 100 ~ 103 | ($CDCl_3$) δ: 1.41(3H,s),1.47(3H,s),2.26(3H,s) 6.68(1H,s),6.90(1H,d,J=2.5Hz), 6.94(1H,d,J=2.5Hz),7.74~7.77 (2H,m),8.11~8.15(2H,m) |
| 31 | 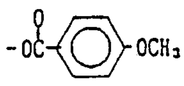 | 〃 | 〃 | oily | ($CDCl_3$) δ: 1.39(3H,s),1.46(3H,s),2.25(3H,s) 3.86(3H,s),6.67(1H,s), 6.87~6.94(4H,m),7.26~8.00 (2H,m) |
| 32 | 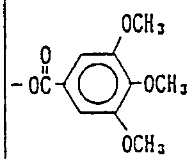 | 〃 | 〃 | 〃 | ($CDCl_3$) δ: 1.41(3H,s),1.48(3H,s),2.26(3H,s) 3.88(6H,s),3.90(3H,s),6.66(1H,s) 6.89(1H,d,J=2.5Hz), 6.93(1H,d,J=2.5Hz), 7.28(2H,s) |
| 33 | 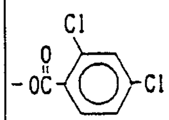 | 〃 | 〃 | 〃 | ($CDCl_3$) δ: 1.40(3H,s),1.45(3H,s),2.26(3H,s) 6.66(1H,s),6.87(1H,d,J=2.5Hz), 6.92(1H,d,J=2.5Hz),7.26~7.31 (1H,m),7.48(1H,d,J=2.0Hz), 7.79(1H,d,J=8.5Hz) |
| 34 | 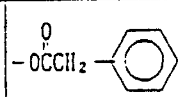 | 〃 | 〃 | 〃 | ($CDCl_3$) δ: 1.24(3H,s),1.29(3H,s),2.22(3H,s) 3.65(2H,s),6.42(1H,s),6.81 (1H,d,J=2.5Hz),6.90 (1H,d,J=2.5Hz),7.24~7.33(5H,m) |
| 35 | 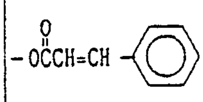 | 〃 | 〃 | 81 ~ 83 | ($CDCl_3$) δ: 1.37(3H,s),1.42(3H,s),2.26(3H,s) 6.43(1H,d,J=15.8Hz),6.59(1H,s), 6.87(1H,d,J=2.5Hz),6.92 (1H,d,J=2.5Hz),7.36~7.40(3H,m) 7.51~7.55(2H,m),7.55 (1H,d,J=15.8Hz) |
| 36 | $-O\overset{O}{\overset{\|}{C}}C_2H_5$ | 〃 | 〃 | oily | ($CDCl_3$) δ: 1.17(3H,t,J=7.4Hz),1.33(3H,s) 1.34(3H,s),2.25(3H,s),2.38 (2H,q,J=7.4Hz),6.45(1H,s),6.83 (1H,d,J=2.0Hz),6.90 (1H,d,J=2.0Hz) |
| 37 | $-O\overset{O}{\overset{\|}{C}}CH_2CH_2CH_3$ | 〃 | 〃 | 〃 | ($CDCl_3$) δ: 0.96(3H,t,J=7.4Hz),1.33(3H,s), 1.34(3H,s),1.67(2H,m,J=7.4Hz), 2.24(3H,s),2.33(2H,q,J=7.4Hz), 6.46(1H,s),6.83(1H,d,J=2.0Hz), 6.90(1H,d,J=2.0Hz) |
| 38 | $-O\overset{O}{\overset{\|}{C}}\underset{Cl}{CH}CH_3$ | 〃 | 〃 | 〃 | ($CDCl_3$) δ: 1.35(3H,s),1.38(3H,s),1.71 (3H,d,J=6.9Hz),2.25(3H,s),4.41 (2H,q,J=6.9Hz),6.46(1H,s), 6.86(1H,d,J=2.5Hz),6.92 (1H,d,J=2.5Hz) |

Table 1 (Continued)

| Compound No. | $R^1$ | $R^2$ | $R^3$ | mp (℃) | NMR Spectra |
|---|---|---|---|---|---|
| 39 | $-O\overset{O}{\overset{\|}{C}}(CH_2)_8-CH_3$ | H | $-CH_3$ | oily | ($CDCl_3$) δ: 0.87(3H, t, J=6.4Hz), 1.2~1.4 (12H, m), 1.32(3H, s), 1.34(3H, s), 1.5~1.7(2H, m), 2.24(3H, s), 2.3~2.4(2H, m), 6.45(1H, s), 6.83 (1H, d, J=2.0Hz), 6.90 (1H, d, J=2.0Hz) |
| 40 | 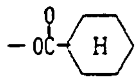 | 〃 | 〃 | 〃 | ($CDCl_3$) δ: 1.2~2.0(10H, m), 1.32(3H, s), 1.34 (3H, s), 2.24(3H, s), 2.3~2.4 (1H, m), 6.45(1H, s), 6.82 (1H, d, J=2.5Hz), 6.90 (1H, d, J=2.5Hz) |
| 41 | $-O\overset{O}{\overset{\|}{C}}CH_2OCH_3$ | 〃 | 〃 | 57 ~ 59 | ($CDCl_3$) δ: 1.32(3H, s), 1.35(3H, s), 2.25(3H, s) 2.6~2.8(4H, m), 3.66(3H, s), 6.46(1H, s), 6.83(1H, d, J=2.4Hz), 6.90(1H, d, J=2.4Hz) |
| 42 | $-O\overset{O}{\overset{\|}{C}}-\underset{CH_3}{\overset{CH_3}{C}}-CH_3$ | 〃 | 〃 | oily | ($CDCl_3$) δ: 1.20(9H, s), 1.34(3H, s), 1.35(3H, s) 2.24(3H, s), 6.43(1H, s), 6.83 (1H, d, J=2.5Hz), 6.69 (1H, d, J=2.5Hz) |
| 43 | $-O\overset{O}{\overset{\|}{C}}CH_2CH_2\overset{O}{\overset{\|}{C}}OCH_3$ | 〃 | 〃 | 74 ~ 70 | ($CDCl_3$) δ: 1.32(3H, s), 1.35(3H, s), 2.25(3H, s) 2.6~2.8(4H, m), 3.66(3H, s), 6.46(1H, s), 6.83(1H, d, J=2.4Hz), 6.90(1H, d, J=2.4Hz) |
| 44 | $-O\overset{O}{\overset{\|}{C}}OCH_3$ | 〃 | 〃 | oily | ($CDCl_3$) δ: 1.33(3H, s), 1.40(3H, s), 2.25(3H, s) 3.85(3H, s), 6.29(1H, s), 6.83 (1H, d, J=2.5Hz), 6.90 (1H, d, J=2.5Hz) |
| 45 | 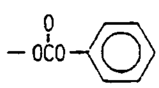 | 〃 | 〃 | 〃 | ($CDCl_3$) δ: 1.36(3H, s), 1.48(3H, s), 2.29(3H, s) 6.37(1H, s), 6.87(1H, d, J=2.5Hz), 6.93(1H, d, J=2.5Hz) |
| 46 | 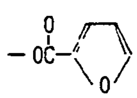 | 〃 | 〃 | 91 ~ 93 | ($CDCl_3$) δ: 1.38(3H, s), 1.44(3H, s), 2.26(3H, s) 6.52(1H, q, J=3.5Hz), 6.63(1H, s), 6.87(1H, d, J=2.0Hz), 6.91 (1H, d, J=2.0Hz), 7.20 (1H, d, J=3.5Hz), 7.60(1H, s) |
| 47 | 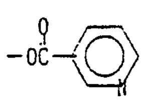 | H | 〃 | oily | ($CDCl_3$) δ: 1.41(3H, s), 1.48(3H, s), 2.26(3H, s) 6.70(1H, s), 7.3~7.5(1H, m), 8.2~8.3(1H, m), 8.80 (1H, d, J=4.5Hz), 9.21(1H, s) |

Table 1 (Continued)

| Compound No. | $R^1$ | $R^2$ | $R^3$ | mp (℃) | NMR Spectra |
|---|---|---|---|---|---|
| 48 | 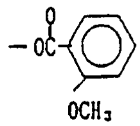 | H | $-CH_3$ | oily | ($CDCl_3$) δ: 1.39(3H,s),1.46(3H,s),2.25(3H,s) 3.86(3H,s),6.67(1H,S),6.85~6.98 (4H,m),7.49(1H,m),7.82(1H,m) |
| 49 | 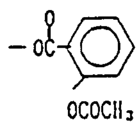 | 〃 | 〃 | 〃 | ($CDCl_3$) δ: 1.38(3H,s),1.44(3H,s),2.09(3H,s) 2.27(3H,s),6.65(1H,S),6.87 (1H,d,J=2.5Hz),6.92 (1H,d,J=2.5Hz),7.10 (1H,d,J=7.9Hz),7.28(1H,m),7.59 (1H,m),7.98(1H,dd,J=6.4Hz,1.5Hz) |
| 50 | 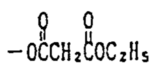 | 〃 | 〃 | 〃 | ($CDCl_3$) δ: 1.23(3H,t,J=7.0Hz),1.33(3H,s), 1.36(3H,s),2.25(3H,s),3.42(2H,s) 4.18(2H,q,J=7.0Hz),6.47(1H,s), 6.83(1H,d,J=2.0Hz),6.91 (1H,d,J=2.0Hz) |
| 51 | $-OC_2H_5$ | 〃 | 〃 | 〃 | ($CDCl_3$) δ : 1.25(3H,s),1.26(3H,m),1.28(3H,s) 2.23(3H,s),3.63(2H,m),5.23(1H,s) 6.70(1H,d,J=2.5Hz),6.83 (1H,d,J=2.5Hz) |
| 52 | 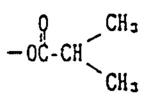 | 〃 | 〃 | 〃 | ($CDCl_3$) δ: 1.16~1.21(6H,m),1.33(3H,s), 1.35(3H,s),2.25(3H,s),2.51~2.62 (1H,m),6.43(1H,s),6.82 (1H,d,J=2.5Hz),6.89 (1H,d,J=2.5Hz) |
| 53 | 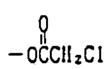 | 〃 | 〃 | 〃 | ($CDCl_3$) δ: 1.35(3H,s),1.38(3H,s),2.26(3H,s) 4.08(2H,s),6.46(1H,s),6.84 (1H,d,J=2.0Hz),6.91 (1H,d,J=2.0Hz) |
| 54 | 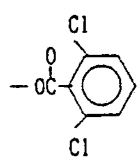 | 〃 | 〃 | 〃 | ($CDCl_3$) δ: 1.40(3H,s),1.44(3H,s),2.27(3H,s) 6.70(1H,s),6.84(1H,d,J=2.5Hz), 6.90(1H,d,J=2.5Hz),7.24~7.37 (3H,m) |

Then, formulation examples and test examples for the herbicidal activity of herbicides according to the present invention are described below.

FORMULATION EXAMPLES AND TEST EXAMPLES

Then, formulation examples and test examples for the herbicidal activity of herbicides according to the present invention are described below.

Formulation Example 1 (Wettable Powder)

A wettable powder was prepared by sufficiently pulverizing and mixing 20 parts by weight of the compound (1) of the present invention, 2 parts by weight of Neopelex (trade name of product: sodium

dodecylbenzene sulfonate manufactured by Kao Atlas Co.), 2 parts by weight of Neugen EA80 (trade name of products: polyoxyethylene nonyl phenyl ether, manufactured by Sanyo Kasei Co.), 5 parts by weight of white carbon and 71 parts by weight of Zieklite (trade name of product: silicic sand powder manufactured by Kokuho Kogyo Co.).

Formation Example 2 (Dust)

A dust was prepared by sufficiently mixing and pulverizing 3 parts by weight of the compound (3) according to the present invention, 0.5 parts by weight of Emulgen 910 (trade name of product: polyoxyethylene nonyl phenyl ether, manufactured by Kao Co.) and 96.5 parts by weight of Sinyo clay (trade name of product: agalmatolite clay manufactured by Asada Seifun Co.)

Formulation Example 3 (Granule)

After sufficinetly mixing 3 parts by weight of finely powderized compound (2) according to the present invention, 2 parts by weight of Neopelex, 2 parts by weight of Sunex P252 (trade name of product: sodium lignin sulfonate manufactured by Sanyo Kokusaku Pulp Co.), 70 parts by weight of Sanritsuberit (trade name of product: bentonite manufactured by Sanritsu Kogyo Co.) and 23 parts by weight of Sanritsutalc (trade name of products: manufactured by Sanritsu Kogyo Co.), they were wetted with addition of an appropriate amount of water and then extruded from a small injection molding machine for pelletization. After air drying and crushing at 30 - 60° C, they were conditioned by a granulator into 0.3 - 2 mm size to obtain granules.

Formulation Example 4 (Emulsifiable Concentrate)

An emulsifiable concentrate was prepared by mixing and dissolving 10 parts by weight of the compound (3) according to the present invention, 10 parts by weight of Solpol 800A (trade name of product: a mixture of non- ionic surface active agent and anionic surface active agent, manufactured by Toho Kagaku Co.) and 80 parts by weight of o-xylene.

Formulation Example 5 (Wettable Powder)

A wettable powder was prepared by sufficiently pulverizing and mixing 20 parts by weight of the compound (18) according to the present invention, 2 parts by weight of Neopelex (trade name of product manufactured by Kao Atlas Co.), 2 parts by weight of Neugen EA80 (trade name of product manufactured by Sanyo Kasei Co.), 5 parts by weight of Carplex (trade name of product manufactured by Shionogi Seiyaku Co.) and 71 parts by weight of Zieklite (trade name of product manufactured by Kokuho Kogyo Co.).

Formulation Example 6 (Wettable Powder)

A wettable powder was prepared by sufficiently pulverizing and mixing 40 parts by weight of the compound (19) according to the present invention, 5 parts by weight of white carbon, 4 parts by weight of ammonium polyoxyethylene alkylphenyl ether sulfate, 2 parts by weight of sodium lignin sulfonate and 49 parts by weight of diatomaceous earth.

Formulation Example 7 (Dust)

A dust was prepared by sufficiently mixing and pulverizing 3 parts by weight of the compound (16) according to the present invention, 0.5 parts by weight of Emulgen 910 (trade name of product manufactured by Kao Co.) and 96.5 parts by weight of Sinyoclay (trade name of product manufactured by Asada Seifun Co.).

Formulation Example 8 (Granule)

After sufficiently mixing 3 parts by weight of finely powderized compound (20) according to the present invention, 2 parts by weight of Neopelex, 2 parts by weight of Sunex 252 (trade name of product manufactured by Sanyo Kokusaku Pulp Co), 70 parts by weight of Sanritsubent (trade name of product manufactured by Sanritsu Kogyo Co.) and 23 parts by weight of Sanritsutalc (trade name of product manufactured by Sanritsu Kogyo Co.), they were wetted with addition of an appropriate amount of water and then extruded to pelletize by a small injection molding machine. After air drying and crushing them at a temperature from 30 to 60 ° C, they were granulated by a granulator into 0.3 - 2 mm size to obtain granules.

Formulation Example 9 (Emulsifiable Concentrate)

An emulsifiable concentrate was prepared by mixing and dissolving 10 parts by weight of the compound (20) according to the present invention, 10 parts by weight of Solpol 800A (trade name of product manufactured by Toho Kagaku Co.) and 80 parts by weight of o-xylene.

Test Example 1 Flooded soil treatment test (pre-emergence treatment)

Soils were packed into each of 1/5000 are Wagner pots, to which seeds or tuber roots of Echinochloa crus-galli, Scirpus juncoides, Eleocharis acicularis and Cyperus serotiuns were seeded and the pot was put into a flooded condition. Previously grown seedlings of paddy field rice (2 - 3 leaf stage) were gathered collected each by two into one hill and two hills were transplanted into each pot and caused to grow in a greenhouse. One day after (before emergence of weeds) they were treated by using the granule prepared from a predetermined amount of the tested compound in accordance with the method as described in the Formulation Example 8, and the state of emergence of weeds and the state of phytotoxicity to the paddy rice plant was observed and investigated after 30 days. The results are shown in Table 2.

In the table, the degree of injury to the tested plants and the degree of phytotoxicity to crops were indicated based on the following standards by the growing state of the plants in comparison with a not-treated case.

| Indication | Growing rate (%) shown by the ratio of air-dry weight based on not-treated lot |
|---|---|
| 5 | 0 - 5 (dead) |
| 4 | 6 - 10 (severe damage) |
| 3 | 11 - 40 (medium damage) |
| 2 | 41 - 70 (less damage) |
| 1 | 71 - 90 (slight damage) |
| 0 | 91 - 100 (no damage) |

The comparative compounds A, B, C, D and E represent the following compounds (also in Test Examples 2 - 3).

A:

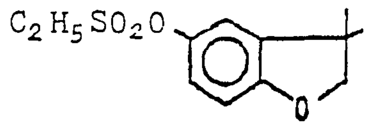

(Compound described in USP-4,162,154)

B:

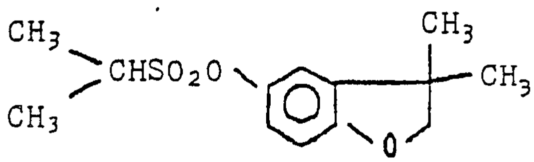

(Compound described in USP-4,162,154)

C:

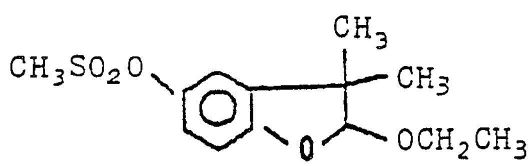

(Compound described in USP-3,689,507)

D:

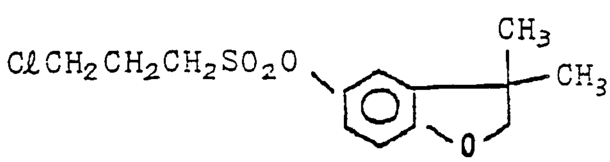

(Compound described in USP-4,162,154)

E:

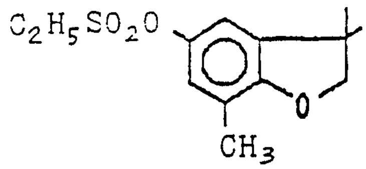

(Compound described in USP-4,162,154)

F:

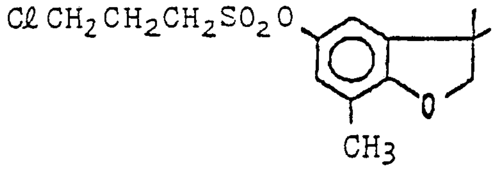

(Compound described in USP-4,162,154)

G:

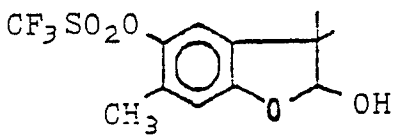

Table 2: Test in Flooded Soil

(Pre-emergence treatment)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 1 | 3 | 4 | 4 | 5 | 5 | 0 |
| | 1 | 4 | 4 | 5 | 4 | 0 |
| | 0.5 | 3 | 3 | 3 | 2 | 0 |
| 2 | 3 | 5 | 5 | 5 | 5 | 1 |
| | 1 | 4 | 4 | 4 | 4 | 0 |
| | 0.5 | 3 | 2 | 3 | 2 | 0 |
| 3 | 3 | 4 | 4 | 5 | 5 | 0 |
| | 1 | 4 | 4 | 4 | 4 | 0 |
| | 0.5 | 2 | 3 | 2 | 4 | 0 |
| 4 | 3 | 4 | 4 | 5 | 5 | 1 |
| | 1 | 4 | 4 | 4 | 4 | 0 |
| | 0.5 | 3 | 2 | 3 | 2 | 0 |
| 5 | 3 | 5 | 5 | 5 | 5 | 1 |
| | 1 | 5 | 4 | 5 | 4 | 0 |
| | 0.5 | 3 | 3 | 3 | 3 | 0 |
| 6 | 3 | 5 | 5 | 5 | 5 | 0 |
| | 1 | 2 | 3 | 2 | 3 | 0 |
| | 0.5 | 0 | 0 | 0 | 0 | 0 |
| 7 | 3 | 5 | 5 | 5 | 5 | 1 |
| | 1 | 5 | 5 | 4 | 4 | 0 |
| | 0.5 | 3 | 3 | 3 | 3 | 0 |
| 8 | 3 | 5 | 5 | 5 | 5 | 0 |
| | 1 | 3 | 3 | 3 | 3 | 0 |
| | 0.5 | 2 | 3 | 2 | 2 | 0 |
| 9 | 3 | 5 | 5 | 5 | 4 | 1 |
| | 1 | 3 | 3 | 3 | 3 | 0 |
| | 0.5 | 2 | 2 | 1 | 1 | 0 |
| 10 | 3 | 5 | 5 | 5 | 5 | 0 |
| | 1 | 5 | 5 | 5 | 4 | 0 |
| | 0.5 | 3 | 3 | 3 | 3 | 0 |

Table 2: (Continued)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 11 | 3 | 4 | 5 | 4 | 4 | 0 |
| | 1 | 3 | 4 | 3 | 3 | 0 |
| | 0.5 | 2 | 3 | 3 | 2 | 0 |
| 12 | 3 | 5 | 5 | 5 | 5 | 0 |
| | 1 | 5 | 5 | 4 | 4 | 0 |
| | 0.5 | 3 | 3 | 3 | 3 | 0 |
| 13 | 3 | 5 | 5 | 5 | 5 | 1 |
| | 1 | 5 | 4 | 4 | 4 | 0 |
| | 0.5 | 3 | 2 | 3 | 2 | 0 |
| 14 | 3 | 5 | 5 | 5 | 5 | 1 |
| | 1 | 5 | 5 | 5 | 5 | 0 |
| | 0.5 | 3 | 3 | 3 | 3 | 0 |
| 15 | 3 | 5 | 5 | 5 | 5 | 1 |
| | 1 | 5 | 5 | 5 | 5 | 0 |
| | 0.5 | 4 | 4 | 5 | 4 | 0 |
| 16 | 3 | 5 | 5 | 5 | 5 | 1 |
| | 1 | 5 | 5 | 5 | 5 | 1 |
| | 0.5 | 5 | 5 | 5 | 5 | 0 |
| 17 | 3 | 5 | 5 | 5 | 5 | 1 |
| | 1 | 4 | 5 | 4 | 4 | 1 |
| | 0.5 | 3 | 4 | 3 | 4 | 0 |
| 18 | 3 | 5 | 5 | 5 | 5 | 0 |
| | 1 | 5 | 5 | 5 | 5 | 0 |
| | 0.5 | 4 | 4 | 4 | 4 | 0 |
| 19 | 3 | 5 | 5 | 5 | 5 | 1 |
| | 1 | 5 | 5 | 5 | 5 | 0 |
| | 0.5 | 5 | 5 | 5 | 5 | 0 |
| 20 | 3 | 5 | 5 | 5 | 5 | 1 |
| | 1 | 5 | 5 | 5 | 5 | 0 |
| | 0.5 | 4 | 5 | 4 | 4 | 0 |

Table 2: (Continued)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 21 | 3<br>1<br>0.5 | 5<br>5<br>5 | 5<br>5<br>5 | 5<br>5<br>5 | 5<br>5<br>5 | 0<br>0<br>0 |
| 22 | 3<br>1<br>0.5 | 5<br>5<br>4 | 5<br>5<br>4 | 5<br>5<br>4 | 5<br>5<br>4 | 1<br>0<br>0 |
| 23 | 3<br>1<br>0.5 | 5<br>5<br>2 | 5<br>4<br>3 | 5<br>5<br>2 | 5<br>4<br>2 | 1<br>0<br>0 |
| 24 | 3<br>1<br>0.5 | 5<br>4<br>2 | 5<br>4<br>2 | 5<br>4<br>3 | 5<br>4<br>2 | 0<br>0<br>0 |
| 25 | 3<br>1<br>0.5 | 5<br>5<br>5 | 5<br>5<br>5 | 5<br>5<br>5 | 5<br>5<br>5 | 1<br>0<br>0 |
| 26 | 3<br>1<br>0.5 | 5<br>5<br>5 | 5<br>5<br>5 | 5<br>5<br>5 | 5<br>5<br>5 | 0<br>0<br>0 |
| 27 | 3<br>1<br>0.5 | 5<br>5<br>5 | 5<br>5<br>5 | 5<br>5<br>5 | 5<br>5<br>5 | 1<br>0<br>0 |
| 28 | 3<br>1<br>0.5 | 5<br>5<br>5 | 5<br>5<br>5 | 5<br>5<br>5 | 5<br>5<br>5 | 1<br>0<br>0 |
| 29 | 3<br>1<br>0.5 | 5<br>5<br>5 | 5<br>5<br>5 | 5<br>5<br>5 | 5<br>5<br>5 | 0<br>0<br>0 |
| 30 | 3<br>1<br>0.5 | 5<br>5<br>5 | 5<br>5<br>5 | 5<br>5<br>5 | 5<br>5<br>5 | 1<br>0<br>0 |

Table 2: (Continued)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 31 | 3 | 5 | 5 | 5 | 5 | 1 |
| | 1 | 5 | 5 | 5 | 5 | 0 |
| | 0.5 | 5 | 5 | 5 | 5 | 0 |
| 32 | 3 | 5 | 5 | 5 | 5 | 0 |
| | 1 | 5 | 5 | 5 | 5 | 0 |
| | 0.5 | 5 | 5 | 5 | 5 | 0 |
| 33 | 3 | 5 | 5 | 5 | 5 | 0 |
| | 1 | 5 | 5 | 5 | 5 | 0 |
| | 0.5 | 5 | 5 | 5 | 5 | 0 |
| 34 | 3 | 5 | 5 | 5 | 5 | 1 |
| | 1 | 5 | 5 | 5 | 5 | 0 |
| | 0.5 | 5 | 5 | 5 | 5 | 0 |
| 35 | 3 | 5 | 5 | 5 | 5 | 1 |
| | 1 | 5 | 5 | 5 | 5 | 0 |
| | 0.5 | 5 | 5 | 5 | 5 | 0 |
| 36 | 3 | 5 | 5 | 5 | 5 | 1 |
| | 1 | 5 | 5 | 5 | 5 | 0 |
| | 0.5 | 5 | 5 | 5 | 5 | 0 |
| 37 | 3 | 5 | 5 | 5 | 5 | 1 |
| | 1 | 5 | 5 | 5 | 5 | 0 |
| | 0.5 | 5 | 5 | 5 | 5 | 0 |
| 38 | 3 | 5 | 5 | 5 | 5 | 1 |
| | 1 | 5 | 5 | 5 | 5 | 0 |
| | 0.5 | 5 | 5 | 5 | 5 | 0 |
| 39 | 3 | 5 | 5 | 5 | 5 | 0 |
| | 1 | 5 | 5 | 5 | 5 | 0 |
| | 0.5 | 5 | 5 | 5 | 5 | 0 |
| 40 | 3 | 5 | 5 | 5 | 5 | 1 |
| | 1 | 5 | 5 | 5 | 5 | 0 |
| | 0.5 | 5 | 5 | 5 | 5 | 0 |

Table 2: (Continued)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 41 | 3<br>1<br>0.5 | 5<br>5<br>5 | 5<br>5<br>5 | 5<br>5<br>5 | 5<br>5<br>5 | 1<br>0<br>0 |
| 42 | 3<br>1<br>0.5 | 5<br>5<br>5 | 5<br>5<br>5 | 5<br>5<br>5 | 5<br>5<br>5 | 0<br>0<br>0 |
| 43 | 3<br>1<br>0.5 | 5<br>5<br>5 | 5<br>5<br>5 | 5<br>5<br>5 | 5<br>5<br>5 | 0<br>0<br>0 |
| 44 | 3<br>1<br>0.5 | 5<br>5<br>5 | 5<br>5<br>5 | 5<br>5<br>5 | 5<br>5<br>5 | 1<br>0<br>0 |
| 45 | 3<br>1<br>0.5 | 5<br>5<br>5 | 5<br>5<br>5 | 5<br>5<br>5 | 5<br>5<br>5 | 1<br>0<br>0 |
| 46 | 3<br>1<br>0.5 | 5<br>5<br>5 | 5<br>5<br>5 | 5<br>5<br>5 | 5<br>5<br>5 | 1<br>0<br>0 |
| 47 | 3<br>1<br>0.5 | 5<br>5<br>5 | 5<br>5<br>5 | 5<br>5<br>5 | 5<br>5<br>5 | 1<br>0<br>0 |
| 48 | 3<br>1<br>0.5 | 5<br>5<br>5 | 5<br>5<br>5 | 5<br>5<br>5 | 5<br>5<br>5 | 1<br>0<br>0 |
| 49 | 3<br>1<br>0.5 | 5<br>5<br>4 | 5<br>5<br>4 | 5<br>5<br>4 | 5<br>5<br>4 | 0<br>0<br>0 |
| 50 | 3<br>1<br>0.5 | 5<br>5<br>5 | 5<br>5<br>5 | 5<br>5<br>5 | 5<br>5<br>5 | 1<br>0<br>0 |

Table 2: (Continued)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 51 | 3<br>1<br>0.5 | 5<br>4<br>4 | 5<br>4<br>3 | 5<br>4<br>3 | 5<br>4<br>4 | 0<br>0<br>0 |
| 52 | 3<br>1<br>0.5 | 5<br>5<br>4 | 5<br>5<br>4 | 5<br>5<br>5 | 5<br>5<br>4 | 0<br>0<br>0 |
| 53 | 3<br>1<br>0.5 | 5<br>5<br>5 | 5<br>5<br>5 | 5<br>5<br>5 | 5<br>5<br>5 | 1<br>0<br>0 |
| 54 | 3<br>1<br>0.5 | 5<br>4<br>3 | 5<br>4<br>3 | 5<br>4<br>3 | 5<br>4<br>3 | 0<br>0<br>0 |
| Comparative Compound A | 3<br>1<br>0.5 | 5<br>3<br>2 | 5<br>4<br>2 | 5<br>5<br>3 | 5<br>5<br>4 | 4<br>2<br>1 |
| Comparative Compound B | 3<br>1<br>0.5 | 5<br>3<br>2 | 5<br>4<br>2 | 5<br>5<br>3 | 5<br>5<br>4 | 3<br>2<br>1 |
| Comparative Compound C | 3<br>1<br>0.5 | 5<br>2<br>0 | 4<br>3<br>0 | 4<br>3<br>0 | 4<br>3<br>0 | 3<br>1<br>0 |
| Comparative Compound D | 3<br>1<br>0.5 | 4<br>2<br>0 | 4<br>3<br>1 | 4<br>2<br>0 | 4<br>3<br>0 | 2<br>0<br>0 |
| Comparative Compound E | 3<br>1<br>0.5 | 4<br>3<br>1 | 3<br>2<br>0 | 3<br>2<br>1 | 4<br>2<br>0 | 1<br>0<br>0 |
| Comparative Compound F | 3<br>1<br>0.5 | 3<br>0<br>0 | 3<br>0<br>0 | 2<br>0<br>0 | 1<br>0<br>0 | 0<br>0<br>0 |
| Comparative Compound G | 3<br>1<br>0.5 | 2<br>0<br>0 | 3<br>1<br>0 | 2<br>0<br>0 | 1<br>0<br>0 | 0<br>0<br>0 |

Test Example 2 Flooded Soil Treatment Test (Growing Stage Treatment)

Soils were packed into each of 1/5000 are Wagner pots to which seeds or tuber roots of Echinochloa crus-galli, Scirpus juncoides, Eleocharis acicularis and Cyperus serotinus were seeded and the pot was put into a flooded condition. Previously grown seedlings of paddy field rice (2 - 3 leaf stage) were gathered each by two into one hill and two hills were transplanted into each pot and caused to grow in a greenhouse. When barnyardgrass grew into the 2-leaf stage, treatment was applied by using the granule prepared from a predetermined amount of the tested compound in accordance with the method as described in the Formulation Example 4, and the state of emergence of weeds and the state of phytotoxicity to the paddy rice plant was observed and investigated after 30 days. The results are shown in Table 3.

In the table, the degree of injury to the tested plants and the degree of phytotoxicity to the crops were indicated in the same manner as in Test Example 1.

Test Example 3 Phytotoxicity Test

Soils were packed into each of 1/500 are Wagner pots and the pot was put into a flooded condition in a greenhouse, to which three previously grown seedlings of paddy

Table 3: Test in Flooded Soil
(Post-emergence treatment)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 1 | 3 | 5 | 4 | 5 | 5 | 0 |
| | 1 | 5 | 4 | 4 | 5 | 0 |
| | 0.5 | 3 | 4 | 3 | 2 | 0 |
| 2 | 3 | 5 | 5 | 5 | 5 | 1 |
| | 1 | 5 | 4 | 4 | 4 | 0 |
| | 0.5 | 2 | 2 | 2 | 2 | 0 |
| 3 | 3 | 5 | 5 | 5 | 5 | 0 |
| | 1 | 4 | 4 | 4 | 4 | 0 |
| | 0.5 | 3 | 2 | 2 | 3 | 0 |
| 4 | 3 | 5 | 4 | 5 | 5 | 1 |
| | 1 | 4 | 4 | 4 | 4 | 0 |
| | 0.5 | 2 | 2 | 2 | 2 | 0 |
| 5 | 3 | 5 | 5 | 5 | 5 | 1 |
| | 1 | 5 | 5 | 5 | 4 | 0 |
| | 0.5 | 3 | 3 | 3 | 2 | 0 |
| 6 | 3 | 4 | 5 | 5 | 4 | 0 |
| | 1 | 3 | 3 | 2 | 3 | 0 |
| | 0.5 | 0 | 0 | 0 | 0 | 0 |
| 7 | 3 | 5 | 5 | 5 | 5 | 1 |
| | 1 | 4 | 5 | 5 | 4 | 0 |
| | 0.5 | 3 | 3 | 3 | 3 | 0 |
| 8 | 3 | 4 | 5 | 4 | 4 | 0 |
| | 1 | 3 | 3 | 3 | 3 | 0 |
| | 0.5 | 2 | 2 | 2 | 1 | 0 |
| 9 | 3 | 5 | 5 | 5 | 5 | 1 |
| | 1 | 3 | 4 | 3 | 3 | 0 |
| | 0.5 | 1 | 1 | 1 | 1 | 0 |
| 10 | 3 | 5 | 5 | 5 | 5 | 0 |
| | 1 | 5 | 5 | 4 | 4 | 0 |
| | 0.5 | 3 | 3 | 3 | 3 | 0 |

Table 3: (Continued)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 11 | 3 | 4 | 4 | 4 | 4 | 0 |
| | 1 | 3 | 4 | 3 | 3 | 0 |
| | 0.5 | 2 | 2 | 2 | 2 | 0 |
| 12 | 3 | 5 | 5 | 5 | 5 | 0 |
| | 1 | 5 | 4 | 4 | 4 | 0 |
| | 0.5 | 3 | 3 | 2 | 3 | 0 |
| 13 | 3 | 5 | 5 | 5 | 5 | 1 |
| | 1 | 5 | 4 | 4 | 5 | 0 |
| | 0.5 | 3 | 2 | 3 | 3 | 0 |
| 14 | 3 | 5 | 5 | 5 | 5 | 1 |
| | 1 | 4 | 5 | 4 | 5 | 0 |
| | 0.5 | 3 | 3 | 3 | 3 | 0 |
| 15 | 3 | 5 | 5 | 5 | 5 | 1 |
| | 1 | 4 | 5 | 5 | 5 | 0 |
| | 0.5 | 4 | 4 | 5 | 4 | 0 |
| 16 | 3 | 5 | 5 | 5 | 5 | 1 |
| | 1 | 5 | 5 | 5 | 5 | 0 |
| | 0.5 | 5 | 5 | 5 | 5 | 0 |
| 17 | 3 | 5 | 5 | 5 | 5 | 1 |
| | 1 | 4 | 5 | 4 | 4 | 0 |
| | 0.5 | 2 | 4 | 3 | 3 | 0 |
| 18 | 3 | 5 | 5 | 5 | 5 | 0 |
| | 1 | 5 | 5 | 4 | 5 | 0 |
| | 0.5 | 4 | 4 | 4 | 4 | 0 |
| 19 | 3 | 5 | 5 | 5 | 5 | 1 |
| | 1 | 5 | 5 | 5 | 5 | 0 |
| | 0.5 | 5 | 5 | 5 | 5 | 0 |
| 20 | 3 | 5 | 5 | 5 | 5 | 1 |
| | 1 | 5 | 5 | 5 | 5 | 0 |
| | 0.5 | 4 | 4 | 4 | 4 | 0 |

Table 3: (Continued)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 21 | 3 | 5 | 5 | 5 | 5 | 0 |
| | 1 | 5 | 5 | 5 | 5 | 0 |
| | 0.5 | 5 | 5 | 5 | 5 | 0 |
| 22 | 3 | 5 | 5 | 5 | 5 | 1 |
| | 1 | 5 | 5 | 5 | 5 | 0 |
| | 0.5 | 5 | 4 | 4 | 4 | 0 |
| 23 | 3 | 5 | 5 | 5 | 5 | 1 |
| | 1 | 5 | 5 | 4 | 5 | 0 |
| | 0.5 | 3 | 3 | 3 | 2 | 0 |
| 24 | 3 | 5 | 5 | 5 | 5 | 0 |
| | 1 | 4 | 4 | 4 | 4 | 0 |
| | 0.5 | 2 | 3 | 2 | 2 | 0 |
| 25 | 3 | 5 | 5 | 5 | 5 | 1 |
| | 1 | 5 | 5 | 5 | 5 | 0 |
| | 0.5 | 5 | 5 | 5 | 5 | 0 |
| 26 | 3 | 5 | 5 | 5 | 5 | 0 |
| | 1 | 5 | 5 | 5 | 5 | 0 |
| | 0.5 | 5 | 4 | 5 | 5 | 0 |
| 27 | 3 | 5 | 5 | 5 | 5 | 1 |
| | 1 | 5 | 5 | 5 | 5 | 0 |
| | 0.5 | 5 | 5 | 5 | 5 | 0 |
| 28 | 3 | 5 | 5 | 5 | 5 | 0 |
| | 1 | 5 | 5 | 5 | 5 | 0 |
| | 0.5 | 5 | 5 | 5 | 5 | 0 |
| 29 | 3 | 5 | 5 | 5 | 5 | 0 |
| | 1 | 5 | 5 | 5 | 5 | 0 |
| | 0.5 | 5 | 5 | 5 | 5 | 0 |
| 30 | 3 | 5 | 5 | 5 | 5 | 1 |
| | 1 | 5 | 5 | 5 | 5 | 0 |
| | 0.5 | 5 | 5 | 5 | 5 | 0 |

Table 3: (Continued)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 31 | 3<br>1<br>0.5 | 5<br>5<br>5 | 5<br>5<br>5 | 5<br>5<br>5 | 5<br>5<br>5 | 1<br>0<br>0 |
| 32 | 3<br>1<br>0.5 | 5<br>5<br>5 | 5<br>5<br>5 | 5<br>5<br>5 | 5<br>5<br>5 | 0<br>0<br>0 |
| 33 | 3<br>1<br>0.5 | 5<br>5<br>5 | 5<br>5<br>5 | 5<br>5<br>5 | 5<br>5<br>5 | 0<br>0<br>0 |
| 34 | 3<br>1<br>0.5 | 5<br>5<br>5 | 5<br>5<br>5 | 5<br>5<br>5 | 5<br>5<br>5 | 0<br>0<br>0 |
| 35 | 3<br>1<br>0.5 | 5<br>5<br>5 | 5<br>5<br>5 | 5<br>5<br>5 | 5<br>5<br>5 | 1<br>0<br>0 |
| 36 | 3<br>1<br>0.5 | 5<br>5<br>5 | 5<br>5<br>5 | 5<br>5<br>5 | 5<br>5<br>5 | 1<br>0<br>0 |
| 37 | 3<br>1<br>0.5 | 5<br>5<br>5 | 5<br>5<br>5 | 5<br>5<br>5 | 5<br>5<br>5 | 1<br>0<br>0 |
| 38 | 3<br>1<br>0.5 | 5<br>5<br>5 | 5<br>5<br>4 | 5<br>5<br>5 | 5<br>5<br>5 | 1<br>0<br>0 |
| 39 | 3<br>1<br>0.5 | 5<br>5<br>5 | 5<br>5<br>4 | 5<br>5<br>5 | 5<br>5<br>5 | 0<br>0<br>0 |
| 40 | 3<br>1<br>0.5 | 5<br>5<br>5 | 5<br>5<br>5 | 5<br>5<br>5 | 5<br>5<br>5 | 1<br>0<br>0 |

Table 3: (Continued)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 41 | 3<br>1<br>0.5 | 5<br>5<br>5 | 5<br>5<br>5 | 5<br>5<br>5 | 5<br>5<br>5 | 1<br>0<br>0 |
| 42 | 3<br>1<br>0.5 | 5<br>5<br>5 | 5<br>5<br>4 | 5<br>5<br>5 | 5<br>5<br>5 | 0<br>0<br>0 |
| 43 | 3<br>1<br>0.5 | 5<br>5<br>5 | 5<br>5<br>4 | 5<br>5<br>5 | 5<br>5<br>4 | 0<br>0<br>0 |
| 44 | 3<br>1<br>0.5 | 5<br>5<br>5 | 5<br>5<br>5 | 5<br>5<br>5 | 5<br>5<br>5 | 1<br>0<br>0 |
| 45 | 3<br>1<br>0.5 | 5<br>5<br>5 | 5<br>5<br>5 | 5<br>5<br>5 | 5<br>5<br>5 | 1<br>0<br>0 |
| 46 | 3<br>1<br>0.5 | 5<br>5<br>5 | 5<br>5<br>5 | 5<br>5<br>5 | 5<br>5<br>5 | 1<br>0<br>0 |
| 47 | 3<br>1<br>0.5 | 5<br>5<br>5 | 5<br>5<br>5 | 5<br>5<br>5 | 5<br>5<br>5 | 1<br>0<br>0 |
| 48 | 3<br>1<br>0.5 | 5<br>5<br>5 | 5<br>5<br>5 | 5<br>5<br>5 | 5<br>5<br>5 | 1<br>0<br>0 |
| 49 | 3<br>1<br>0.5 | 5<br>5<br>4 | 5<br>4<br>3 | 5<br>5<br>4 | 5<br>5<br>4 | 0<br>0<br>0 |
| 50 | 3<br>1<br>0.5 | 5<br>5<br>5 | 5<br>5<br>5 | 5<br>5<br>5 | 5<br>5<br>5 | 1<br>0<br>0 |

Table 3: (Continued)

| Conpound No. | Dosage (kg/ha) | Echinochloa crus-galli | Monochoria vaginais | Scripus juncaides | Cyperus difformis | Transplanted paddy rice plant |
|---|---|---|---|---|---|---|
| 51 | 3 | 5 | 5 | 5 | 5 | 0 |
| | 1 | 4 | 4 | 4 | 4 | 0 |
| | 0.5 | 3 | 3 | 3 | 4 | 0 |
| 52 | 3 | 5 | 5 | 5 | 5 | 1 |
| | 1 | 5 | 4 | 5 | 5 | 0 |
| | 0.5 | 4 | 3 | 5 | 4 | 0 |
| 53 | 3 | 5 | 5 | 5 | 5 | 1 |
| | 1 | 5 | 5 | 5 | 5 | 0 |
| | 0.5 | 5 | 5 | 5 | 5 | 0 |
| 54 | 3 | 5 | 5 | 5 | 5 | 0 |
| | 1 | 4 | 4 | 4 | 3 | 0 |
| | 0.5 | 2 | 2 | 3 | 3 | 0 |
| Comparative Compound A | 3 | 5 | 4 | 5 | 5 | 3 |
| | 1 | 3 | 3 | 4 | 4 | 2 |
| | 0.5 | 2 | 2 | 3 | 4 | 0 |
| Comparative Compound B | 3 | 5 | 4 | 5 | 5 | 3 |
| | 1 | 3 | 3 | 4 | 4 | 2 |
| | 0.5 | 2 | 2 | 3 | 4 | 1 |
| Comparative Compound C | 3 | 4 | 3 | 4 | 4 | 3 |
| | 1 | 2 | 2 | 3 | 2 | 0 |
| | 0.5 | 0 | 1 | 0 | 0 | 0 |
| Comparative Compound D | 3 | 4 | 4 | 4 | 4 | 2 |
| | 1 | 2 | 2 | 2 | 3 | 0 |
| | 0.5 | 0 | 0 | 0 | 0 | 0 |
| Comparative Compound E | 3 | 4 | 4 | 4 | 4 | 0 |
| | 1 | 3 | 0 | 1 | 2 | 0 |
| | 0.5 | 0 | 0 | 0 | 0 | 0 |
| Comparative Compound F | 3 | 2 | 3 | 2 | 1 | 0 |
| | 1 | 0 | 1 | 0 | 0 | 0 |
| | 0.5 | 0 | 0 | 0 | 0 | 0 |
| Comparative Compound G | 3 | 3 | 4 | 2 | 1 | 0 |
| | 1 | 1 | 0 | 0 | 0 | 0 |
| | 0.5 | 0 | 0 | 0 | 0 | 0 |

field rice plant (2 - 3 leaf stage) were transplanted. Seven days after, they were treated by using granule prepared from a predetermined amount of the tested compound in accordance with the method as described in the Formulation Example 4. The pot was conditioned with water leakage of 0.5 cm/day for 10 days after the treatment and, then with no water leakage. Growing state of the paddy field rice was observed and examined after 30 days. The results are shown in Table 4.

In the table, the growing state of the paddy field rice was investigated by measuring the leaf length, number of stalks and air-dry weight and indicated by "%" in comparison with a not-treated lot.

As shown in Tables 2 - 4 above, the compounds according to the present invention show extremely less or no substantial phytotoxicity to the paddy field rice plants and can be used safely to paddy field rice although they have higher herbicidal effect as compared with the compounds A, B and C. Further, they had higher herbicidal effect even compared with the compound D. Further, compound E having the methyl group on the 6-position showed no substantial activity.

The compounds according to the present invention have advantageous features of showing high herbicidal effect as the herbicide, particularly, high herbicidal effect against important weeds such as barnyardgrass, water nutgrass, bulrush, etc. although the phytotoxicity to the paddy field rice plant is remarkably reduced as compared with the compound known by literatures. That is, the compounds according to the present invention have a high applicability as a paddy field rice herbicide and the present invention can provide an extremely useful herbicide.

Table 4: Result in Phytotoxicity

| Compound No. | Dosage (kg/ha) | Plant Length(%) | Number of staks(%) | Air-dry weight(%) |
|---|---|---|---|---|
| 1 | 3 | 102 | 100 | 98 |
| 2 | 3 | 98 | 95 | 95 |
| 3 | 3 | 105 | 107 | 105 |
| 4 | 3 | 95 | 90 | 90 |
| 5 | 3 | 102 | 98 | 100 |
| 6 | 3 | 102 | 100 | 102 |
| 7 | 3 | 98 | 102 | 99 |
| 8 | 3 | 107 | 98 | 105 |
| 9 | 3 | 98 | 95 | 95 |
| 10 | 3 | 100 | 98 | 98 |
| 11 | 3 | 97 | 105 | 102 |
| 12 | 3 | 98 | 100 | 100 |
| 13 | 3 | 103 | 105 | 103 |
| 14 | 3 | 97 | 93 | 96 |
| 15 | 3 | 96 | 94 | 97 |
| 16 | 3 | 92 | 102 | 96 |
| 17 | 3 | 90 | 95 | 92 |
| 18 | 3 | 102 | 110 | 104 |
| 19 | 3 | 107 | 97 | 103 |
| 20 | 3 | 97 | 100 | 100 |
| 21 | 3 | 105 | 107 | 104 |

Table 4: (Continued)

| Compound No. | Dosage (kg/ha) | Plant Length(%) | Number of staks(%) | Air-dry weight(%) |
|---|---|---|---|---|
| 22 | 3 | 98 | 103 | 99 |
| 23 | 3 | 95 | 98 | 97 |
| 24 | 3 | 100 | 103 | 101 |
| 25 | 3 | 103 | 105 | 104 |
| 26 | 3 | 98 | 100 | 100 |
| 27 | 3 | 108 | 105 | 106 |
| 28 | 3 | 107 | 104 | 105 |
| 29 | 3 | 105 | 107 | 105 |
| 30 | 3 | 102 | 100 | 100 |
| 31 | 3 | 95 | 97 | 95 |
| 32 | 3 | 100 | 103 | 102 |
| 33 | 3 | 112 | 105 | 107 |
| 34 | 3 | 98 | 102 | 101 |
| 35 | 3 | 102 | 98 | 100 |
| 36 | 3 | 95 | 98 | 98 |
| 37 | 3 | 100 | 103 | 101 |
| 38 | 3 | 105 | 107 | 105 |
| 39 | 3 | 99 | 102 | 100 |
| 40 | 3 | 102 | 95 | 98 |
| 41 | 3 | 97 | 95 | 97 |

Table 4: (Continued)

| Compound No. | Dosage (kg/ha) | Plant Length(%) | Number of staks(%) | Air-dry weight(%) |
|---|---|---|---|---|
| 42 | 3 | 105 | 100 | 100 |
| 43 | 3 | 102 | 100 | 101 |
| 44 | 3 | 97 | 92 | 95 |
| 45 | 3 | 96 | 94 | 95 |
| 46 | 3 | 102 | 98 | 101 |
| 47 | 3 | 99 | 102 | 100 |
| 48 | 3 | 97 | 94 | 95 |
| 49 | 3 | 107 | 102 | 103 |
| 50 | 3 | 92 | 100 | 98 |
| 51 | 3 | 93 | 105 | 102 |
| 52 | 3 | 103 | 105 | 105 |
| 53 | 3 | 95 | 92 | 93 |
| 54 | 3 | 107 | 102 | 103 |
| Comparatve Compound A | 3 | 65 | 35 | 42 |
| Comparatve Compound B | 3 | 77 | 52 | 58 |
| Comparatve Compound C | 3 | 75 | 45 | 51 |
| Comparatve Compound D | 3 | 82 | 75 | 75 |
| Comparatve Compound E | 3 | 89 | 100 | 91 |
| Comparatve Compound F | 3 | 95 | 103 | 97 |

## Claims

1. A herbicidal 2,3-dihydrobenzofuran of the formula:

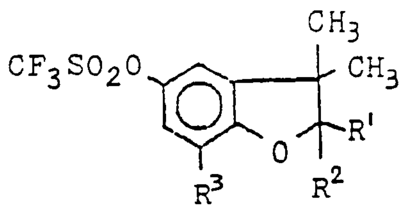

( I )

wherein $R^1$ represents a hydrogen atom, a hydroxy group, a lower alkoxy group, an amino group, a morpholino group or an esterified group: $-OCOR^4$ (wherein $R^4$ represents a lower alkyl group, a cycloalkyl group, a halogen-substituted lower alkyl group, a lower alkoxy-substituted lower alkyl group, or a group: $-(CH_2)_nCO_2R^5$ (wherein $R^5$ represents a lower alkyl group and n represents the integer 1 or 2), a benzyl group, a styryl group, a lower alkoxy group, a phenoxy group, a pyridyl group, a thienyl group, furyl group, or a group:

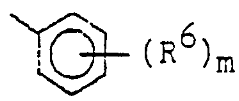

(wherein $R^6$ represents a hydrogen atom, a halogen atom, a cyano group, an acetoxy group, a lower alkyl group, a lower alkoxy group and m represents an integer from 1 to 5)), $R^2$ represents a hydrogen atom or together with $R^1$ represents an oxygen atom, and $R^3$ represents a hydrogen atom, a methyl group or a halogen atom.

2. A 2,3-dihydrobenzofuran of claim 1, wherein $R^2$ is hydrogen atom.

3. A 2,3-dihydrobenzofuran of claim 1, wherein $R^3$ is methyl.

4. A 2,3-dihydrobenzofuran of claim 1, wherein $R^1$ is an esterified hydroxy group as defined therein, hydroxy, morpholino, or collectively with $R^2$ an oxygen atom.

5. A herbicide containing in admixture with carrier, a herbicidally effective amount of 2,3-dihydrofuran of the formula:

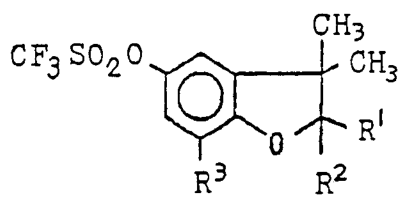

( I )

wherein $R^1$ represents a hydrogen atom, a hydroxy group, a lower alkoxy group, an amino group, a morpholino group or an esterified group: $-OCOR^4$ (wherein $R^4$ represents a lower alkyl group, a cycloalkyl group, a halogen-substituted lower alkyl group, a lower alkoxy-substituted lower alkyl group, or a group: $-(CH_2)_nCO_2R^5$ (wherein $R^5$ represents a lower alkyl group and n represents the integer 1 or 2), a benzyl group, a styryl group, a lower alkoxy group, a phenoxy group, a pyridyl group, a thienyl group, furyl group, or a group:

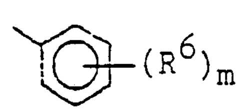

(wherein $R^6$ represents a hydrogen atom, a halogen atom, a cyano group, an acetoxy group, a lower alkyl

group, a lower alkoxy group and m represents an integer from 1 to 5)), $R^2$ represents a hydrogen atom or together with $R^1$ represents an oxygen atom, and $R^3$ represents a hydrogen atom, a methyl group or a halogen atom.

6. A herbicide containing in admixture with carrier, a herbicidally effective amount of 2,3-dihydrofuran of claim 2.

7. A herbicide containing in admixture with carrier, a herbicidally effective amount of 2,3-dihydrofuran of claim 3.

8. A herbicide containing in admixture with carrier, a herbicidally effective amount of 2,3-dihydrofuran of claim 4.

9. A process for producing a compound represented by the general formula (II):

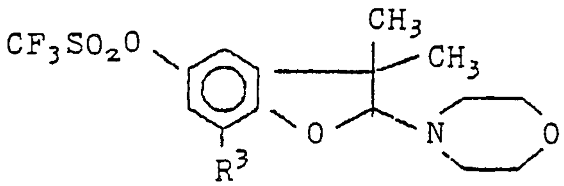

( II )

where $R^3$ represents a hydrogen atom, methyl group or halogen atom, wherein a compound represented by the general formula (III):

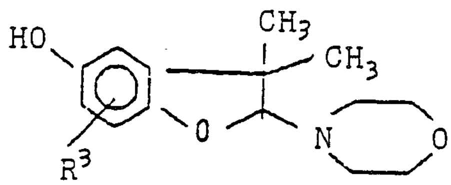

( III )

where $R^3$ has the same meanings as described above, and a trifluoromethane sulfonyl halide or trifluoromethane sulfonic acid anhydride are brought into reaction and then separated and purified.

10. A process for producing a compound represented by the general formula (IV) :

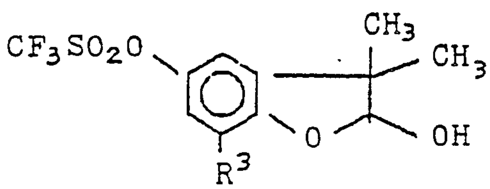

( IV )

where $R^3$ represents a hydrogen atom, methyl group or halogen atom, wherein a compound represented by the general formula (II) :

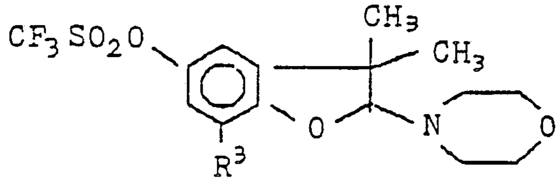

( II )

where $R^3$ has the same meanings as described above is hydrolyzed.

11. A process for producing a compound represented by the general formula (V):

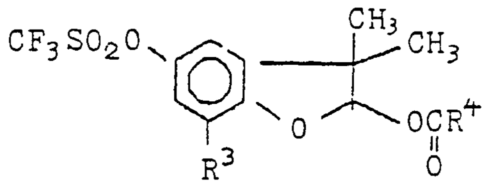

( V )

where $R^3$ represents a hydrogen atom, methyl group or halogen atom, $R^4$ represents a lower alkyl group, cyclo alkyl group, halogen-substituted lower alkyl group, lower alkoxy-substituted lower alkyl group, a group: $-(CH_2)_nCO_2R^5$ ( wherein $R^5$ represents a lower alkyl group and n represents the integer 1 or 2), benzyl group, styryl group, lower alkoxy group, phenoxy group, pyridyl group, thienyl group, furyl group, or a group:

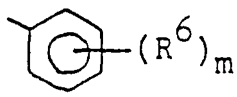

(wherein $R^6$ represents a hydrogen atom, halogen atom, cyano group, acetoxy group, lower alkyl group, lower alkoxy group and m represents an integer from 1 to 5), wherein a compound represented by the general formula (IV):

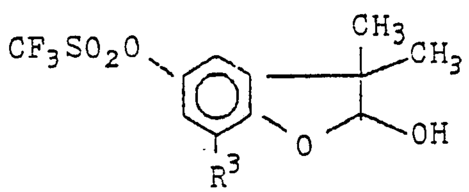

( IV )

where $R^3$ represents the same meanings as described above and a compound represented by the general formula $R^4COX$ ( wherein X represents a halogen atom and $R^4$ has the same meanings as described above) or by the general formula: $(R^4CO)_2O$ (wherein $R^4$ has the same meanings as described above) are brought into reaction.

12. A process for producing a compound represented by the general formula (VI):

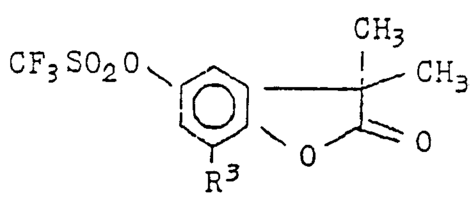

( VI )

where $R^3$ represents a hydrogen atom, methyl group or halogen atom, wherein a compound represented by the general formula (IV):

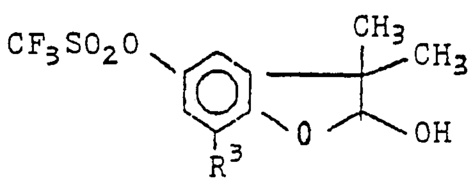

( IV )

where $R^3$ has the same meanings as described above, is oxidized.

13. A process for producing a compound represented by the general formula (VII):

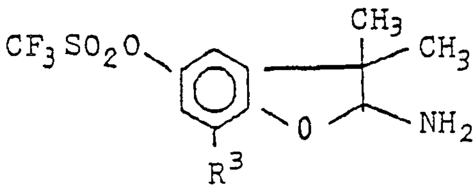

(VII)

where $R^3$ represents a hydrogen atom, methyl group or halogen atom, wherein a compound represented by the general formula (II):

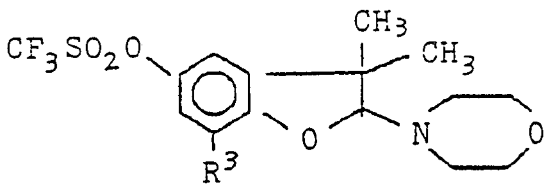

(II)

where $R^3$ has the same meanings as described above is brought into reaction with ammonia.

14. A process for producing a compound represented by the general formula (VIII):

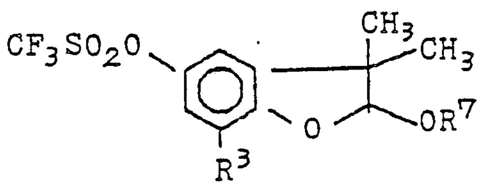

(VIII)

where $R^3$ represents a hydrogen atom, methyl group or halogen atom, and $R^7$ represents a lower alkyl group, wherein a compound represented by the general formula (II):

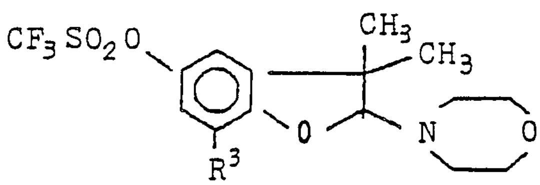

(II)

where $R^3$ has the same meanings as described above is brought into reaction with a compound represented by the general formula $R^7OH$ (wherein $R^7$ represents the same meanings as described above).

15. A process for producing a compound represented by the general formula (IX):

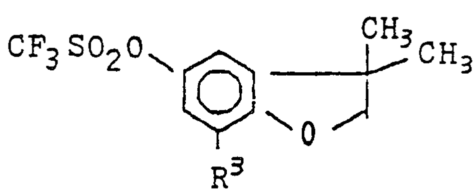

(IX)

where $R^3$ represents a hydrogen atom, methyl group or halogen atom, wherein a compound represented by the general formula (X) :

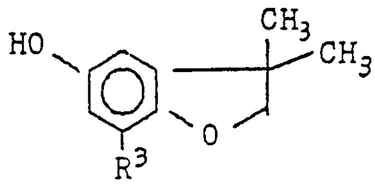

( X )

where R[3] has the same meanings as described above is brought into reaction with trifluoromethane sulfonyl halide or trifluoromethane fulfonic acid anhydride.